(19) Europäisches Patentamt
European Patent Office
Office européen des brevets

(11) EP 2 052 080 B1

(12) **EUROPEAN PATENT SPECIFICATION**

(45) Date of publication and mention of the grant of the patent:
**06.08.2014 Bulletin 2014/32**

(21) Application number: **07790098.3**

(22) Date of filing: **24.07.2007**

(51) Int Cl.:
*C12N 15/29* (2006.01) *C12N 15/53* (2006.01)
*C12N 9/02* (2006.01) *C12N 15/82* (2006.01)

(86) International application number:
**PCT/IN2007/000308**

(87) International publication number:
**WO 2008/020454 (21.02.2008 Gazette 2008/08)**

(54) **RESISTANCE OF PLANTS TO BIOTIC AND ABIOTIC STRESSES BY OVEREXPRESSION OF PROTOCHLOROPHYLLIDE OXIDOREDUCTASE C AND ITS ISOFORMS**

RESISTENZ VON PFLANZEN GEGEN BIOTISCHEN UND ABIOTISCHEN STRESS DURCH ÜBEREXPRESSION VON PROTOCHLOROPHYLLID-OXIDOREDUKTASE C UND IHREN ISOFORMEN

RÉSISTANCE DES PLANTES AUX STRESS BIOTIQUES ET ABIOTIQUES PAR LA SUREXPRESSION DE LA PROTOCHLOROPHYLLIDE OXIDORÉDUCTASE C ET DE SES ISOFORMES

(84) Designated Contracting States:
**DE FR GB**

(30) Priority: **17.08.2006 IN DE18432006**

(43) Date of publication of application:
**29.04.2009 Bulletin 2009/18**

(73) Proprietors:
- **Department of Biotechnology New Delhi 110 003 (IN)**
- **Jawahar Lal Nehru University New Dehli 110 067 (IN)**

(72) Inventor: **TRIPATHY, Baishnab Charan New Delhi 110 067 (IN)**

(74) Representative: **Radkov, Stoyan Atanassov Urquhart-Dykes & Lord LLP The Podium 1 Eversholt Street London NW1 2DN (GB)**

(56) References cited:
- **SPERLING U ET AL: "OVEREXPRESSION OF LIGHT-DEPENDENT PORA OR PORB IN PLANTS DEPLETED OF ENDOGENEOUS POR BY FAR-RED LIGHT ENHANCES SEEDLING SURVIVAL IN WHITE LIGHT AND PROTECTS AGAINST PHOTOOXIDATIVE DAMAGE" PLANT JOURNAL, BLACKWELL SCIENTIFIC PUBLICATIONS, OXFORD, GB LNKD- DOI:10.1046/J.1365-313X. 1997.00649.X, vol. 12, no. 3, 1 January 1997 (1997-01-01), pages 649-658, XP009001267 ISSN: 0960-7412**
- **DATABASE BIOSIS [Online] BIOSCIENCES INFORMATION SERVICE, PHILADELPHIA, PA, US; February 2007 (2007-02), TRIPATHY B ET AL: "Overexpression of protochlorophyllide oxidoreductase C results in tolerance to singlet oxygen-mrdiated oxidative stress in plants" XP009139232 Database accession no. PREV200700527743 & PHOTOSYNTHESIS RESEARCH, vol. 91, no. 2-3, February 2007 (2007-02), page 317, 14TH INTERNATIONAL CONGRESS OF PHOTOSYNTHESIS; GLASGOW, UK; JULY 22 27, 2007 ISSN: 0166-8595**
- **OOSAWA N. ET AL.: 'Identification and light-induced expression of a novel gene of NADPH-protochlorophyllide oxireductase isoform in Arabidopsis thaliana' FEBS LETTERS vol. 474, no. 2-3, pages 133 - 136, XP004337194**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 052 080 B1



(Cont. next page)

- FRICK G. ET AL.: 'An Arabidopsis porB porC double mutant lacking light-independent NADPH: protochlorophyllide oxidoreductases B and C is highly chlorophyll-deficient and developmentally arrested' PLANT J. vol. 35, no. 2, July 2003, pages 141 - 153, XP003018756
- MASUDA T. ET AL.: 'Functional analysis of isoforms of NADPH: protochlorophyllide oxidoreductase (POR), PORB and PORC, in Arabidopsis thaliana' PLANT CELL PHYSIOL. vol. 44, no. 10, 2003, pages 963 - 974, XP008102361
- HOLTORF S ET AL: "COMPARISON OF DIFFERENT CONSTITUTIVE AND INDUCIBLE PROMOTERS FOR THE OVEREXPRESSION OF TRANSGENES IN ARABIDOPSIS THALIANA", PLANT MOLECULAR BIOLOGY, SPRINGER, DORDRECHT, NL, vol. 29, 1 November 1995 (1995-11-01), pages 637-646, XP002036874, ISSN: 0167-4412, DOI: 10.1007/BF00041155

## Description

FIELD OF INVENTION:

The invention relates to construction of plant transformation vector for over expression of Protochlorophyllide oxidoreductase C and transformation of plants with the said vector.

BACKGROUND OF THE INVENTION:

Plants are vulnerable to biotic and abiotic stresses and they have a better potential of plant productively and quality than that being currently achieved. Resistance to oxidative stress is also another Important aspect.

The gene protochlorophyllide oxidoreductase was cloned in Japan, Switzerland and in the inventor *slab In Indian and have been published. There is no state of art in the over expression of Protochlorophyllide oxidoreductase C.

Sperling *et al*., 1997 (D1) describes experiments where over expression of light dependent PORA or PORB in plants depleted of an endogenous (PRO) by far red light enhances seedling survival in white light and protects against photooxidative damage.

They test the hypothesis by analysing the in vivo phenotypes resulting from constitutive over expression of either PORA or PORB in transgenic arabidopsis seedlings, comprising depleted seedlings of endogenous (PRO) by growth of cFR.

Tripathy *et al*., (D2) is directed to experiments where over expression of porC leads to tolerance of singlet oxygen mediated oxidative stress in plants.

An object of the invention is to construct plant transformation vector for over expression of protochlorophyllide oxidoreductase C.

Another object is to construct plant transformation vector for over expression of protochlorophyllide oxidoreductase C and transform plant, preferably Arabidopsis by the said transformation vector.

Further object is to construct plant transformation vector for over expression of protochlorophyllide oxidoreductase C and produce plants resistant to oxidative stress, abiotic stress and biotic stress by over expression of protochlorophyllide oxidoreductase C.

Another object is to construct plant transformation vector for over expression of protochlorophyllide oxidoreductase C and produce transgenic plants which under high light, high or low temperatures, high salinity or soil pH, drought, water lagging, UVB radiation, bacterial, fungal or viral infections, become resistant to stress and produce higher biomass, have higher harvest index than their wild type counterparts.

Yet another object to minimize the generation of singlet oxygen by reducing the concentration of the tetrapyrrolic photosensitizer protochlorophyllide.

Still another object to control plant death induced by oxidative stress by regulating the steady state accumulation of chlorophyll biosynthetic intermediates and generation of singlet oxygen.

BRIEF DESCRIPTION OF THE INVENTION:

According to this invention a process for the construction of plant transformation vector comprising:

- digesting a binary vector pCAMBIA1304 with EcoRI and Sal I;
- subjecting the digested binary vector to the step of end filling by Klenow;
- re-ligating the said binary vector by known method;
- obtaining CaMV 35 s promoter cassette with Ω enhancer from pSH9; incorporating the said Camv35S prometer cassette into the Hind III site of the said binary vector to obtain modified pCAMBIA 1304 vetor;
- subjecting pore DNA fragment (1206 bp) to the step of amplification using a pair of primers;
- introducing EcoRI restriction sites to both the primers;
- ligating the said amplified cDNA fragment to pGEM T-easy;
- extracting EcoRI digested porC cDNA fragment from cloned pGEM T-easy; inserting said EcoRI digested porC cDNA fragment into modified pCAMBIA 1304 plant transformation vector to produce recombinant plasmids construct (pCAMBIA 1304-AtporC)

BRIEF DESCRIPTION OF THE ACCOMPANYING DRAWINGS:

Fig.1 : shows that wild type and transgenic (porC) plants exposed to high light.

Fig. 2: shows that wild type and transgenic (porC) plants exposed to light induced by different concentrations of oxidative stress inducing sulstance (ALA).

Fig. 3a: shows that omega (Ω) enhancer was used for Agrobacterium mediated plant transformation.

Fig. 3b: shows that Southern blot analysis of different T3 stable Arabidopsis transgenic lines (PORCx).

Fig. 3c: shows that northern and western blot analysis revealed that porC and its gene product were highly over expressed.

Fig. 3d: shows that northern and western blot analysis revealed that porC and its gene product were highly over expressed.

Fig. 4a: shows that the over expression of porC in Arabidopsis plants resulted in greener plants.

Fig. 4b: shows that PORCx plants resulted in efficient photoconversion of Pchlide to Chlide.

Fig. 4c: shows that $^1O_2$ production and its measurement.

Fig. 5a: shows that light-stressed WT plants.

Fig. 5b: shows that accumulated reduced amounts of anthocyanin.

Fig. 5c: shows that presence of excess $^1O_2$ and low at reduced $^1O_2$ in WT and PORCx plants.

Fig. 5d: shows that reduced membrane lipid peroxidation owing to less production of $^1O_2$.

Fig. 5e: shows that in light-stressed PORCx plants the Fv/Fm ratio was not affected demonstrating that due to reduced $^1O_2$ generation and membrane lipid peroxidation their photosynthetic efficiency was intact.

Fig. 6a: shows that both WT and PORCx plants accumulated excess of Pchlide, Proto IX and MP(E) in ALA-treated WT and PORCx plants in dark.

Fig. 6b: shows that both WT and PORCx plants phototransformed Pchlide to Chlide.

Fig. 6c: shows that PORCx plants due to increased abundance of PORC protein, phototransformation of Pchlide to Chlide was much higher.

Fig. 6d: shows that reduced accumulation of the photosensitizer Pchlide resulted in less production of light-mediated-$^1O_2$ in PORCx plants.

Fig. 6e: shows that the modulation of $^1O_2$ generation in PORCx plants by manipulation of the level of the photosensitizer Pchlide pool led to reduced MDA production.

Fig. 6f: shows that the Fv/Fm ratio was almost unchanged in ALA-treated and light exposed PORCx plants.

Fig. 6g: shows that the PORCx plants survived and produced flowers even after 10 days of light exposure.

Fig. 6h: shows that light-induced death of WT plants whereas PORCx plants survived.

DETAILED DESCRIPTION OF THE INVENTION:

Oxidative stress is the ultimate manifestation of almost all kinds of biotic and abiotic stresses that affect forest and crop ecosystems. Singlet oxygen, one of the reactive oxygen species is produced in pigment bed of the photosynthetic apparatus and via type II photosensitization reactions of tetrapyrroles[3]. It causes oxidative cellular damage and plant death. Unlike detoxification of superoxide by superoxide dismutase there is no enzymatic detoxification mechanism available to destroy singlet oxygen produced from chlorphyll biosynthetic intermediates in light stress. The present invention aims to minimize the generation of singlet oxygen by reducing the concentration of the tetrapyrrolic photosensitizer protochlorophyllide, via genetic approach i.e., manipulation of the protochlorophyllide oxidoreductase contents that photo-transforms protochlorophyllide to chlorophyllide. Here we show that in Arabidopsis thaliana overexpression of porC gene that encodes for the light-inducible isoform of protochlorophyllide oxidoreductase C, resulted in reduced accumulation of the photosensitizer protochlorophyllide and minimal production of singlet oxygen in response to light stress and 5-aminolevulinic acid (ALA) treatment. It is demonstrated that it is possible to control plant death induced by oxidative stress by regulating the steady state accumulation of chlorophyll biosynthetic Intermediates and generation of singlet oxygen.

Regulation of oxidative stress is extremely important to protect plants from high light and other abiotic and biotic stresses. To minimize the generation of $^1O_2$ I manipulated the steady state accumulation of Chi biosynthetic intermediates by producing protochlorophyllide (Pchlide) oxidoreductase C (porC) over expressing (PORCx) plants.

Protochlorophyllide (Pchlide) oxidoreductase (POR), the first light-dependent enzyme in the Chl biosynthesis pathway has three isoforms i.e., PORA, PORB and PORC. porC is light-inducible and its expression increases in high light. To increase PORC amount for efficient photoconversion of Pchlide to chlorophyllide (Chlide) porC was over expressed in A. thaliana (ecotype Col). The modified Pcambia 1304 binary vector containing the porC cDNA, driven by the control of the cauliflower mosaic virus (CaMV) 35S promoter having omega ($\Omega$) enhancer was used for Agrobacterium mediated plant transformation (Fig.3a). Southern blot analysis of different T3 stable Arabidopsis transgenic lines (PORCx) derived from the independent transformation events revealed that they had single copy insertion of the trans-gene (Fig. 3b). Northern and Western blot analysis revealed that porC and its gene product were highly over expressed (4-5 fold as compared to WT) is different PORCx lines (Fig. 3c, d).

The over expression of porC in Arabidopsis plants resulted in greener plants having enhanced Chi contents (22% more in T12 & 27% more in T13) (Fig. 4a), indicating that porC is a limiting enzyme in Chi biosynthesis pathway. There was no change in carotenoid contents of PORCx plants. Increased accumulation of porC transcripts and PORC protein in PORCx plants resulted in efficient photoconversion of Pchlide to Chlide and reduced accumulation of photosensitizer Pchlide under steady state illumination (Fig. 4b). Due to higher metabolic flux, the concentrations of other Chl biosynthetic intermediates i.e., protoporphyrin IX (Proto IX), Mg-protoporphyrin + its monoester (MPE) were also reduced in PORCx plants (Fig. 4b). To examine the light-intensity-dependence of generation of $^1O_2$, both WT and PORCx plants were transferred to either low light (LL) (50 $\mu$moles photons $m^{-2}$ $s^{-1}$ at 21°C) or moderate light (ML) (330 $\mu$moles photons $m^{-}s^{-1}$) in two different Conviron plant growth chambers. ML treatment resulted in increase in temperature to 33°C. The plastidic membranes were isolated from LL-and ML-grown WT and PORCx plants and their $^1O_2$ production was measured (Fig. 4c). Generation of $^1O_2$ was dependent on light intensity,i.e., it was less in LL and much more in ML. However, due to reduced presence of the tetapyrrolic photosensitizers the $^1O_2$ production was minimal in PORCx plants in light-stressed conditions (Fig. 4c).

Light-stressed WT plants were purple (Fig. 5a) due to accumulation of flavonoid anthocyanin. Under identical conditions PORCx plants were green having partially purple lower leaves (Fig. 5a) and accumulated reduced amounts of anthocyanin (Fig. 5b). However, in LL due to reduced generation of $^1O_2$ less amounts of anthocyanin were produced (Fig. 5b) in both WT and PORCx plants. The gene expression of chalcone synthase (CHS) involved in anthocyanin synthesis[17] was also regulated by $^1O_2$ i.e., its expression was more in the presence of excess $^1O_2$ and low at reduced $^1O_2$ in WT and PORCx plants (Fig. 5c). Excess production $^1O_2$ led to generation of high amounts of malondialdehyde (MDA) in light-stressed-WT plants (Fig. 5d). Under identical conditions PORCx plants had reduced membrane lipid peroxidation owing to less production of $^1O_2$ (Fig. 5d). In the same vein in light-stressed PORCx plants the Fv/Fm ratio was not affected demonstrating that due to reduced $^1O_2$ generation and membrane lipid peroxidation their photosynthetic efficiency was intact (Fig. 5e).

To probe further if manipulation of the level of Chl biosynthetic intermediates would lead to control of oxidative stress induced by $^1O_2$, both WT and PORCx plants were treated with ALA (3 mM), the precursor of Chl and heme and were kept in dark overnight (14h). As shown in (Fig. 6a), both WT and PORCx plants accumulated excess of Pchlide, Proto IX and MP(E) in ALA-treated WT and PORCx plants in dark. Immediately after light (100 $\mu$moles photons $m^{-2}$ $s^{-1}$) exposure, both WT and PORCx plants phototransformed Pchlide to Chlide (Fig. 6b). The efficiency of phototransformation of Pchlide to Chlide was 73% in WT plants whereas in PORCx plants due to increased abundance of PORC protein, phototransformation of Pchlide to Chlide was much higher i.e., 93% (Fig. 6c). Reduced accumulation of the photosensitizer Pchlide (Fig. 6b) resulted in less production of light-mediated-$^1O_2$ in PORCx plants (Fig. 6d). The modulation of $^1O_2$ generation in PORCx plants by manipulation of the level of the photosensitizer Pchlide pool led to reduced MDA production (Fig. 6e). The Fv/Fm ratio was almost unchanged in ALA-treated and light exposed (6h) (Fig. 6f) PORCx plants demonstrating that the photosynthetic efficiency was not affected.

Due to oxidative stress there was a severe morphological injury to WT plants. Exposure of ALA-treated (3 mM) and dark incubated (14h) WT plants to light (100 $\mu$moles photons $m^{-2}s^{-1}$) caused wilting of leaves followed by appearance of prominent necrotic patches and subsequent death of plants within 24h (Fig. 6g). However, under identical conditions ALA-treated PORCx plants had partial damage to their leaves. The PORCx plants survived and produced flowers even after 10 days of light exposure (Fig. 6g). The extent of damage to plants was dependent on ALA concentration. Application of 3 mM or 5 mM ALA to WT and PORCx plants resulted in light-induced death of WT plants whereas PORCx

| Name | Sequence |
|---|---|
| PORC | F: 5'tctaga gaa ttc atg gct ctc caa gct gcc tat tct g 3' |
| | R 5' gaa ttc tca tgc caa acc aac aag ctt ctc gc 3' |
| | M 5'ggg gat ccc aga cag tta cag 3' |
| | Int F 5' ggg aat tct caa acc atc tta gag |
| | Int R: 5' cgg gat ccc gct cag tct tct ttg ctc cgg 3' |
| PORB | F: 5' tct aga gaa ttc atg gcc ctt caa gct gtc ctt ctc 3'<br>R 5' gaa ttc tta ggc caa gcc cac gag 3' |
| | Int F: 5'ggg aat tcg cct cca tta ccg acc a |
| | Int R: 5' ggg tcg acg cc g tcc acg gat ttt g 3' |
| PORA | Int F: 5' ggg aat tca ttt cac ttt cgg agc a |
| | Int R: 5' ggg tcg acg cgg tct aag gaa gat t 3' |
| CAO | F: 5' gag ctc gag atg aac gcc gcc gtg ttt agt cc |
| | R: 5' tcg aat tcc tta gcc gga gaa agg tag 3' |
| | M: 5' gag ctc gag atc aag agt caa tgg aga ttg 3' |
| GsaT | Int F: 5' gag cga cac aga gaa gtt tgg 3 |
| | R: 5' cct act cag tac cct ctc agc 3' |
| UroD | Int F: 5' ccg gtg tgg atg ttg tga |
| | R: 5' agt atc atg aat ccg gct tgt g 3' |
| PPX | Int F: 5' gac acg gct aaa tca tct cta ac 3' |
| | R 5' cgg gat cct gtt cag tgg ccg gtg gac ca 3' |
| Fech | Int F 5' ggg agt gta gaa gaa cta tta gc 3' |
| | R 5' gtg gtg act tcg agc acc aag 3' |
| chl I | Int F: 5' gca ctt gca gct ctc gga 3' |
| | R: 5' ggc tgc agt cag ctg aaa atc tcg gca |
| chl D | Int F: 5'gat acc gag aac aag ttt gtt tc 3' |
| | R: 5' cgg gat cca agc ttt gaa caa ttc ttc aga tca g 3' |
| chl H | F 5' gtc tca tga aca cca atc cca ac 3' |
| | R. 5' ggg aat tcg tcg act tat cga tcg atc cct 3' |
| ch!P | F. 5' gca tgg cga cga cgg tta ca 3' |
| | R: 5' gct taa aca cta agc ttc tca atc tc 3' |
| 35S Int | F: 5' ccc act atc ctt cgc aag ac 3' |
| GUS | F. 5' cgc gga tcc atg tta ggt tcg tag aaa ccc c 3' |
| | R: 5' gct cga gct ccc ggg tca ttg gcc tcc ctg ctg 3' |
| | Int R: 5' atg ccc aca ggc cgt cga gtt 3' |
| Kan | F. 5' tcg acc atg ggg att gaa caa gat gg 3' |
| | R: 5' att cga gct ctc aga aga act cgt caa gaa ggc 3' |

(continued)

| Name | Sequence |
|---|---|
| Act I | F. 5' atg gct gat ggt gaa gac att 3' |
| | R: 5' tca gaa gca ctt cct gtg aac a 3' |
| Oligo dT | 5' ttt ttt ttt ttt ttt ttt ttt ttt 3' |
| CHS | F:- 5' atg gtg atg gct ggt gct tc 3' |
| | R: 5' tta gag agg aac gct gtg caa g 3' |
| PORC Promoter | F1: 5' ggc aag ctt ggt ata att ggg ctc aaa aag g 3' |
| | F2: 5'ggc aag ctt gat gag ggg gga aaa agt 3' |
| | F3: 5' ggc aag ctt gta gaa gat gaa gag agc tg 3' |
| | R3 5' ggg gga tcc atc acc gtt gat agg tga act g 3' |
| | F4 5' ggc aag ctt cag atc agt tga gag tta ac 3' |
| | R: 5' ggg gga tcc tgt tgt acg gaa ctg aag gtg c 3' |

plants survived (Fig. 6h). Application of higher concentrations of ALA (10 mM) caused the death of both WT and PORCx plants.

Results demonstrate that reduction in the steady state accumulation of plant tetrapyrroles would limit $^1O^2$ production and cellular damage in light-stressed plants. In the same vein plant death caused by $^1O_2$ generated from over-accumulated Chl biosynthetic intermediates in ALA-treated plants could be minimized by efficiently converting them to Chl. This approach could be exploited to generate PORCx crop plants that would be resistant to the herbicidal treatment of ALA or other compounds that induce oxidative stress and biotic and abiotic stresses for better crop yield.

1. Construction of plant transformation vector for over-expression of pORC in plants:

A binary vector pCAMBIA 1304 was digested with EcoRI and Sal I, end filled by Klenow and then re-ligated using the standard procedure (Sambrook. et. al 1989). A CaMV 35 S promoter cassette along with the Ω enhancer was taken out from pSH9 and inserted into the Hind III site of the binary vector. For the amplification of a porC cDNA fragment (1206 bp) cDNA library of A.thaliana was used. PCR was carried out with a pair of primers as given in table-4. In both the primers EcoRI restriction sites were introduced. The amplified cDNA fragment was ligated to pGEMT-Easy, and then EcoRI digested porC cDNA fragment was taken out from cloned pGEMT-Easy and inserted into the modified pCAMBIA 1304 plant transformation vector.

Transformation of Agrobacterium: Recombinant plasmid construct (pCAMBIA 1304-AtporC) was transformed into Agrobacterium by freeze thaw method. For the preparation of competent cells, Agrobacterium tumefaciens (GV3101) was grown in 50 ml YEM medium (0.04% yeast extract, 1% mannitol, 0.01 NeC1, 0.02% $MgSO_4$, $7H_2O$ and 0.5% $K_2HPO_4$) at 28°C with vigorous shaking until the O.D $_{600}$ reached 0.5 to 0.6. The culture was chilled on ice and centrifuged at 3000 X g for 5 min at 4°C. The pellet was resuspended in 1 ml of ice cold $CaCl_2$ (20mM) and 0.1 ml aliquots were dispensed in pre-chilled eppendorf tubes and stored at-80°C.

Transformation of Agrobacterium with pCAMBIA -porC plasmid construct was carried out by mixing 10 μg of DNA with competent cells followed by immediate freezing in liquid nitrogen. Subsequently cells were thawed, incubating the Eppendorf tubes at 37°C for 5 min and 1 ml of YEM medium was added to the tube and incubated at 28°C for 3 hrs. Cells were spread on a YEM agar plate supplemented with 50 μg/ml kanamycin and 25μg/ml rifampicin and incubated at 28°C. Transformed colonies that appeared after 1-2 days were analysed either by PCR and the positive colonies were confirmed by restriction digestion of the purified recombinant plasmids.

Plant Transformation: One positive colony of Agrobacterium tumefacians from each construct was used to transform Arabidopsis by vacuum infiltration method (Clough and Bent, 1998). A single colony was inoculated into 30 ml of liquid YEM medium with kanamycin plus rifampicin and grown for one day at 28°C. One ml of this bacterial culture was inoculated into fresh medium and grown overnight

Arabidopsis Transformation of by Vacuum infiltration: The plastic pots were filled to top with agropeat soil and covered with required size of mosquito net using rubber bands. The vernalised Arabidopsis seeds at 4°C for 3 days were resuspended in milli-Q water and 20-30 seeds per pot were uniformly distributed over the agropeat soil. The pots were transferred to the growth chamber. Plants were allowed to grow under 16 hrs dark and 8 hrs light till bolts emerged.

Bolts were clipped off to produce multiple secondaries.

Vacuum Infiltration: A single colony of Agrobacterium tumefaciens was inoculated into 25 ml of liquid YEM with kanamycin and rifampicin and grown for 48 hrs at 28°C. One ml of this bacterial culture was inoculated into 1 L of fresh medium and allowed to grow for overnight at 28°C. The cells were harvested at 5000 g for 10 min at room temperature. The pellet was resuspended in infiltration medium (0.5 X MS salts, 1X B5 vitamins, 5% sucrose, 0.04 M BAP, 0.02% silvet, pH 5.7). The plants having multiple secondary bolts were inserted on 250 ml beaker containing Agrobacterium tumefaciens strain with proper construct. The entire set up was kept in bell jar and the vacuum was applied till bubbles were formed on the leaves and stem. The vacuum was rapidly released and plants were left in liquid for 1-2 min. The plants were removed and covered with saran-wrap (to maintain humidity) and left overnight The plants were uncovered after one day and then grown under green house condition. The seeds were harvested after one month.

Seed Plating in Culture Medium: Seeds (40 mg) of Arabidopsis were surface sterilized with 2 ml of sterilization solution [30% v/v bleach (100% bleach contain 5% hypochlorite) with 1 μl/ml of 20% Triton X-100] and gently mixed by inversion for 15 min. The seeds were allowed to settle for 1 or 2 min before decanting the bleach. The tube with seeds was filled with sterile water and mixed. Washing of seeds was repeated several times till the bleach smell disappeared (it normally takes more than 5 washes). The seeds were finally resuspended in required volume of sterile water and then plated on GM medium containing kanamycin for selection of heterozygous transgene. The sterile water was allowed to evaporate in laminar flow hood and transferred plates in cold under dark for 2 days. After 2 days the plates were transferred to light.

The seedlings, which contain heterozygous transgene, survived on kanamycin plates and all other susceptible seedlings were bleached out Resistant seedings were transferred to soil pots and grew them for 3 months to collect the seeds.

This process was continued for 3 to 4 generations to obtain homozygous lines.

II. Methodology used in the whole process:

Plant materials and growth conditions: The Arabidopsis theliana ecotype Columbia-0(Col-0) was grown in soil or on GM medium containing 0.8% agar at 22°C ± 2°C with a photoperiod of 16 hrs light and 8 hrs dark except when specific treatments were given.

General sterilization procedures: Culture media, glassware and tissue culture tools were sterilized by autoclaving at 121°C and 151-p/ inch$^2$ pressure for 15 min. Antibiotics and other heat-labile components used were fitter sterilized using a syringe filtration unit fitted with an autoclaved cellulose nitrate membrane filter of 0.22 μm pore size (mdi, India).

Table 2: Composition of the GM medium (Gamborg et al., 1968)

| Constituents | Concentration (mg/L) |
|---|---|
| 1X MS salt mixture | |
| 1% Sucrose | |
| Inositol | 100 |
| Thiamine | 1.0 |
| Pyridoxine | 0.5 |
| Nicotinic acid | 0.5 |
| MES | 500 |
| Adjusted to pH 5.7 with 1 M KOH | |
| 0.7-0.8% Difco Bacto Agar | |

Table 3: Composition of the MS nutrient medium (Murashige and Skoog, 1962)

| Constituents | Concentration (g/L) |
|---|---|
| **Major salts (20X)** | |
| $NH_4NO_3$ | 33.0 |
| $KNO_3$ | 38.0 |
| $CaCl_2H_2O$ | 8.0 |
| $MgSO_4.7H_2O$ | 7.4 |
| $KH_2PO_4$ | 3.4 |

(continued)

| | |
|---|---|
| **Minor salts (200X)** | |
| $MnSO_4.H_2O$ | 4.46 |
| $H_3BO_3$ | 1.24 |
| $ZnSO_4.7H_2O$ | 1.72 |
| KI | 0.16 |
| $Na_2MoO_4.2H_2O$ | 0.05 |
| $CuSO_4.5H_2O$ | 0.005 |
| $CoCl_2.6H_2O$ | 0.005 |
| **Iron Source (200X)** | |
| $FeSO_4.7H_2O$ | 0.027 |
| $Na_2EDTA$ | 0.037 |
| **Organic Constituent (100X)** | 0.5 |
| Nicotinic Acid | 0.5 |
| Pyridoxine-HCL | 1.0 |
| ThiamineHCl.$H_2O$ | 0.1 |
| Myoinositol | 2.0 |
| Glycine | |

Nutrient Media: For in vitro experiment, the GM medium (Table 2), Gamborg et al., 1968) was used in case of Arabidopsis. The composition of MS medium (Murashige and Skoog, 1962) was mentioned in (Table 3). The stock solutions of various components as given In (Table 3) were prepared in Milli-Q water, stored at 4°C and mixed as required just before use. All the phytohormone stocks were prepared in DMSO and stored at 4°C. The medium was prepared from stock solutions and the pH was adjusted to 5.7 using 1 M NaOH prior to the addition of agar and then autoclaved. The filter-sterilized solution of heat labile antibiotics was added to the autoclave medium pre-cooled to 45°C.

Recombinant DNA techniques for clonning and DNA analysis: Rapid amplification of the cDNA or genomic DNA fragment was carried out using Taq DNA polymerase and a set of convergent primers. Reaction mixture (50 μl) contained 30-50 ng DNA template, 150ng (or 30pmoles) of each primer, 8 μl of 1.25 mM dNTPs, 5 μl 10 X Taq buffer and 2.5 units of Taq polymerase. The reaction condition of PCR consisted of denaturation (94°C), annealing (varies from gene to gene) and extension (72°C) for 1 min per 1kb pair to be amplified. Reaction was continued for 30 cycles to obtain an amplified product. An aliquot from the mix was electrophoresed on 1% agarose gel to check for amplification.

Purification of DNA fragment from Agarose Gel: PCR amplified product or restriction enzyme digested plasmid was electrophoresed on 1% agarose gel. The desired fragment was identified using standard molecular weight marker (1kb ladder or Lambda DNA digested with Hind III) and purified using the following technique.

Qiaqusick/ GFX Gel Extraction Column: To the excised pieces of agarose gel containing DNA fragment 3 volumes of QG/captured buffer (as supplied with Qiaquick/ GFX gel extraction kit) was added and dissolved by heating to 55°C for 10 min. The mixture was loaded onto Qiaquick/GFX spin column and spun briefly. The flow though was discarded and the column was washed with PE/Wash buffer(Olagen GMBH, Amersham pharmacia). The purified DNA fragment was eluted with 50 μl of 10 mM Tris-Cl, pH 8.0.

Preparation of competent cells and transformation: DH5α cells were made competent by following the protocol of Hanahan, 1983. A single colony of DH5a was picked up and inoculated into 5 ml of LB medium and grown overnight at 37°C. One ml of this was inoculated freshly into 100 ml of LB and grown at 37°C till O.D $_{600}$ of 0.4 - 0.5 was reached (2 - 3 hrs). The cells were harvested by centrifugation at 3000 X g for 10 min. The pellet was resuspended in 40 ml of ice cold 100mM $CaCl_2$ solution, incubated on ice for 1 hr, centrifuged and the pellet was resuspended in a 4ml of ice cold 100 mM $CaCl_2$ containing 15% glycerol solution. The cell suspension (0.1 ml) was aliquoted into autoclaved un-opened eppendorf tubes and stored at -80°C.

DNA fragments were ligated to the appropriate vectors by using T4 DNA ligase overnight at 4°C or 16°C. The ligation mixture was added to the competent cells and mixed by tapping and then incubated for 30 min at 4°C. All steps were carried out in a laminar hood under sterile conditions. The cells were subjected to heat shock by incubating at 42°C for 90 sec, allowed to stand for 2 - 5 min on ice followed by addition of 0.9 ml of LB and then grown at 37°C with gentile shaking (185 rpm). Different aliquots of these transformed competent cells were plated onto LB plate containing appropriate antibiotic. For blue white selection, 10 μg IPTG and 1 μg X-gal per plate were spread prior to plating the cells. The plates were incubated overnight at 37°C. Transformed cells containing recombinant plasmid were confirmed by colony PCR and restriction digestion.

Isolation and purification of plasmid DNA: Five milliliters of overnight grown culture of bacterial cells were harvested by centrifugation at a maximum speed for 1 min in a microfuge. The pellet was resuspended in 100 μl of ice-cold solution I (50 mM Tris-HCl, pH 8.0, 10mM EDTA, 100μg/ml RNase A) and vortexed vigorously. Then cells were lysed by adding 200μl freshly prepared solution II (200mM NaOH, 1% SDS) and gently mixed by inverting the tube 5 times and then kept on ice for 5 min.

Following this, 150μl of chilled solution III (3.0 M potassium acetate, pH 5.5) was added and mixed by inversion. This mixture was incubated on ice for 3-5 min and centrifuged at maximum speed in a microfuge for 5 min at 4°C. The supernatant was extracted with equal volumes of Phenol: Chloroform: Iso amyl alcohol (25:24:1) at maximum speed for 2 min at 4°C. The DNA precipitation was carried out by adding 1/10 volume of 3M sodium acetate (pH 52) and two volumes of ethanol (room temperature). The mixture was incubated at room temperature for 2min and centrifuged at maximum speed for 5 min to precipitate DNA. The plasmid DNA was washed with 70% ethanol, vacuum dried and dissolved in minimal volume of water or TE buffer.

Spectrophotometric Estimation of Nucleic acid: The quantity and quality of the nucleic acid was determined by measuring the absorbance at 260 nm and 280nm. The amount was calculated taking 1.0 $A_{260}$=50μg/ml for DNA and 1.0 $A_{250}$ =40μg for RNA. The purity of the nucleic acid was determined by calculating the ratio $A_{260}/A_{280}$ for each sample.

Pchlide Extraction and Purification: Pchlide was extracted according to Hukamani and Tripathy, 1992. Leaves (Incubated over night with 25ml of 4.5 mM ALA and 5mM 2, 2 dipyridil) were homogenized in 200 ml of acetone: 0.1 N $NH_4OH$(9:1). The fully esterified tetrapyrroles were extracted into the hexane while the mono and di-carboxylic tetrapyrroles remained in the hexane extracted acetone residue solvent mixture fraction (HEAR). The pigments were extracted from HEAR fraction to 1/3 volume of diethylether after addition of 1/20 volume of saturated NaC! and 1/40 vol of 0.5M phosphate buffer (pH 7.0). The ether extracts containing the Pchlide along with other carboxylic tetrapyrroles were concentrated by vacuum drying, dried under $N_2$ gas and were dissolved in minimal volume of methanol: acetone=4:1. The pigments dissolved in methanol: acetone solution were chromatographed on thin layers of silica gel H developed in toluene: ethyl acetate: ethanol (4:1:1) at 4°C. Pchlide, which migrated having Rf value of 0.32 was eluted in methanol: acetone (4:1).

Thin Layer Chromatography: 43 g of silica gel (Type H, sigma chemical Co.) was dissolved in 100 ml of distilled water and mixed in a mixer. This solution was spread uniformly over the clean glass plates as a thin layer. Glass plates were then dried in the over at 50°C.

Spectrophotometry: Absorption spectrum was recorded on a Shimadzu UV 260 spectrophotometer. For the calculation of concentrations of pure samples of Pchlide was dissolved in ether, extinction co-efficient of $3.56x10^4M^{-1}cm^{-1}$ at 624 nm was used.

Spectrofluorometry: Fluorescence spectra were recorded in ratio mode in a computer-driven SLM AMINCO 8000 spectrofluorometer having a photon-counting device. Rhodamine B was used in the reference channels as a quantum counter. A tetraphenylbutadiene block was used to adjust the voltage in the sample as well as in the reference channels to 20,000 counts per second at excitation and emission wavelengths of 348 and 422 nm, respectively. To reduce the noise level, the sample channel photomuttiplier tube was cooled to -20°C by a thermoelectric cooling device. The emission spectra were recorded from 600 to 700 nm at excitation and emission bandwidths of 4 nm.

Raising of polyclonal Anti body: Polyclonal anti body against ATPORC was raised In rabbit as per the standard procedure (Herlow and Lane 1988). Two-four months of old New Zealand white rabbits were used for raising antibodies. Before the primary injection, pre-immune serum was collected by bleeding the rabbit by puncturing the eye vein. Primary immunization was carried out by making a emulsion of crushed and homogenized gel pieces containing about 150 μg of respective proteins width complete Freund's adjuvant in equal proportions. After four weeks, a secondary immunization was done with an emulsion of incomplete Freund's adjuvant and 100μg of individual proteins in gel pieces. Test bleeds were done to check the fitter of the antibodies by western analysis. A total number of 5 boosters were given at time interval of 4 weeks and finally blood was collected using capillary from a puncture eye vein. The serum isolated from blood was aliquoted and stored at 70°C.

2-Dimensional Gel Electro phoresis: Protein was estimated by Brodford method (Bradford, 1976) after half hour incubation in ice. Protein sample containing 100μg of protein was added to rehydration buffer (6M urea, 2 M thiourea, 2% CHAPAS, 0.2% ampholyte, 50MDTT).

Isoelectric focuring: Protein sample containing 100 μg in 125 μl of rehydration buffer was spread equally in the lain of rehydration tray. 1PG (Immobile pH gradient) strip of 7 cm (pH 4-7) was laid on the sample facing the gel side down. Mineral oil was over laid to prevent any evaporation. It was left for passive rehydration for 16 hour at 20°C temperature in isoelectric focusing (IEF). After the strips got rehydrated it was moved to IEF focusing tray. Two paper wicks wet with 8μl of milli Q water were put on two electrodes. Focusing tray was adjusted in Isoelectric focusing cell. Mineral oil was overlaid and run was carried out according to program given below. After running got completed 1 pG strips were taken out, put in equilibration buffer 1 for 10 min and subsequently in equilibration buffer II for another 10 min. Then 1 PG strip was kept on stacking gel and 12.5% of SDS-PAGE (sodium dodecyl subfate polyacrylamide gel electrophoresis) was run at constant voltage of 100V. On one side of the gel, medium range molecular weight marker (14.3 kDa tp 97.4 kDa) was loaded. Equilibration buffer I: 6M urea, 2% SDS, 0.375 M Tris-HCL (pH8.8), 20% glycerol and 2% (w/v) DTT. Equilibration buffer II: 6 M urea, 2% SDS, 0.375 M Tris HCL (pH 8.8), 20% glycerol and 2.5% idoacetamide.

Running Programme for Isoelectric Focusing:

| 7cm strip | Voltage | time | Volt-hours | ramp |
|---|---|---|---|---|
| Step 1 | 250 | 20min | - | Unear |
| Step 2 | 4000 | 2 hr | - | Linear |
| Step 3 | 4000 | 2.3hr | 10,000V-hr | Rapid |
| Total | | 5 hr | -14,000 v-hr | |

Silver Straining: After completion of SDS-PAGE, gels were soaked in fixative solution (40% ethanol and 12% glacial acetic acid with 75 μl of formaldehyde) for 1 h with 2-3 changes of the same solution with time interval. Then these were washed threetimes for 20 min each in 50% ethanol. After that gels were treated with 0.02% solution of sodium thiosulphate for 1 min and washed with double distilled water for 1 min. Then the gels were submerged and shook in silver nitrate solution (0.2%) containing 75 μl of formal dehyde for 20 min. After silver nitrate staining gels were washed for 1 min with distilled water, then transferred to 6% sodium carbonate solution and formaldehyde was added drop by drop to it till the desired colour was obtained. The reaction was stopped in the 50% methanol and 12% glacial acetic acid solution. Gels were stored in 5% glacial acetic acid solution. Whole of the staining was done with continuous shaking and the timings were maintained very accurately.

III. Analysis of Transgenic plants:

Gus Histochemical Assay: Putative transgenic plants were screened histochemically for verification of the expression of wid. A gene (Jefferson, 1987). Histochemical array for GUS was carried out in the intact tissues (organ or whole seedlings or free hand cut sections). Tissue from the control and transgenic plants were submerged in fixation buffer (2% formaldehyde, 50mM sodium phosphate pH 7, 0.05% Triton X-100) and Vacuum infiltrated for 4-5 min on ice and kept at room temperature for 10 min. The fixation buffer was removed and the material was washed twice with 50mM sodium phosphate buffer pH 7 to remove fixative buffer. The tissue sample were stained with 1.5mM of X-gluc, 50mM sodium phosphate pH 7 and 0.1 % Triton X-100 by vacuum infiltrating for 5-10 min and then kept the tissue at 37°C overnight in darkness. After staining, tissue was rinsed extensively in ethanol to remove the chlorophyll.

Preparation of Plant Genomic DNA for PCR Analysis: Small piece of leaf tissue (1cm X 1cm) was ground with 400μl of buffer (2% CTAB, 1.4 M Nad, 20 mM EDTA, 100mM Tris-cl and 0.1 % B Mercap to ethanol) and mixed with another 400 μl buffer and kept at 60°C in a water bath for 30 min. The aqueous layer was extracted twice with chloroform: isoamyl alcohol (24:1) and genomi cDNA was precipitated with 2/3 volume of isopropanol for 30 min at-20°C. Centrifuged the samples at maximum speed for 3 min at room temperature. The pellet was washed 3 times with 70% ethanol, dried and dissolved in 30μl TE or sterile water containing 20μg/ml RNase and incubated at 37°C for 30 min.

Southern Blotting: Genomic DNA was digested with different restriction enzymes at 37°C and then electrophoresed overnight on 0.8% agarose gel. Following electrophoresis DNA was visualized by ethidium bromide staining. DNA from the gel was transferred to nylon membrane using the capillary method as described in Sambrook et al., 1989. Briefly, the gel was treated first with depurination solution (0.2 N HC1) for 20 min if the DNA fragments were more than 10 Kb in size followed by 10 min treatment in denaturation solution (1.5 M NaC1, 0.5 N NaOH) and then 2 washes of 10 min each in neutralizing solution (1 M ammonium acetate). The DNA was transferred to nylon membrane (Hybond-N, Amersham, England) by capillary transfer in 10X SSC (1.5 M NaCl, 0.15 M sodium citrate, pH 7.0) or 1 M ammonium acetate for overnight and then the blot was baked for 2 h at 80°C under vacuum. The membrane was pre-hybridized for 4-6 h at 65°C in buffer containing 5X SSC, 5X Denhardt reagent (0.5% Ficoll, 0.5% PVP, 0.5% BSA), 0.1% SDS and 100 μg/ml denatured salmon sperm DNA and 10% Dextran sulphate. Thereafter, to the pre-hybridization solution, radiolabeled probe (pre-denatured by boiling for 10 min) was added. The probe was prepared by using the random-labelling Kit (Amersham, England) and purified using Sephadex G-50 spun column (Sambrook et al., 1989). After 16-18 h of incubation at 65°C in the hybridization solution, membranes were washed once with 2X SSC (room temperature) and twice sequentially in 1X SSC, 0.1% SDS for 15 min each at 65°C.

EXAMPLES:

ANALYSIS AT TRANSCRIPT LEVELS

Treatments for RNA Isolation: Rosette Leaves of young Arabidopsis plants grown for 35-40 days under 16h/8h photoperiod at 22°C were used for extraction of total RNA. Four to five days old etiolated Arabidopsis seedlings were exposed to different quality and quantity of light and after a particular period of time of treatments whole seedlings were used to extract RNA. RNA was also isolated from plants grown in low light (LL), high light (HL) and exposed to herbicide i.e; δ-ALA.

Isolation of Total RNA by TRI reagent (Sigma) Method: About 0.1 g of tissue was homogenized in 1 ml of trizol and the insoluble material was separated from the homogenate by centrifugation at 11,000 g for 10 min at 2-8°C. The supernatant was transferred into a fresh Eppendorf tube and to this 0.2 ml chloroform was added and mixed vigorously. The sample was centrifuged at 12,000 rpm for 15 min at 2-8°C and the supernatant was transferred to a fresh eppendorf tube. The RNA was precipitated from the aqueous phase by mixing with 0.5 ml isopropanol followed by centrifugation at 12,000 rpm for 15 min. The pellet was washed with 80% ethanol dried and dissolved in minimal volume of Rnase A free sterile water.

RT-PCR: Reverse transcription was carried out using 2μg of total RNA and oligo-dT as primer. The RNA was treated with RNAse free DNAse (5unit/μl) to make sure that there is no contamination of DNA. The reaction mixture includes 1mM dNTPs, 0.5μM oligo-dT, 20 U Rnase inhibitor, 10mM DTT, 1x RT buffer and 20-30 unit AMV reverse transcriptase in total volume of 50μl. The reaction mixture was incubated for one hour at 37°C. Using this reaction mixture as template PCR was carried out with gene specific primers to check the expression of the respective genes. To ensure linearity of the reaction, the minimum number of cycles needed to visualize the transcripts was first determined. When the condition for RT-PCR linearity was established, runs were performed and repeated at least for three times using independently treated samples.

Preparation of Probe by Random Primer Libelling Method: Twenty-five ng DNA, 5 μl of primer was taken in an eppendorf. After denaturation by heating to 95-100°C for 5 min, brief spin was given. Then dNTPs (4 μl each) were added without dCTP, which was used as label, 5μl of reaction buffer, 5 μl of $\alpha^{32}$P dCTP and 2μl of enzyme and appropriate volume of water was added to make total volume of 50μl. The contents were mixed gently by pipetting up and down and capped the tube. The reaction mixture was incubated at 37°C for 1 hr and stopped by adding of 5μl of 0.2 EDTA. Before use in a hybridization reaction the labeled DNA was denatured by heating to 100°C for 5 min and chilled on ice.

Northern Hybridisation: Total RNA was fractionated on 1% formaldehyde denaturing agarose gel according to Sambrook et al., 1989. Gel was electrophoresed for 3-4 hrs and after completion of the run, the gel was rinsed in DEPC treated water and equilibrated with 20 X SSC for 10 min. RNA was transferred to nylon membrane (Amersham hybond -N) by capillary transfer in 5 X SSC for 16 hrs. Nylon membrane was rinsed in 6 X SSC, cross-linked in Stratalinker, soaked in 5% acetic acid and stained with methylene blue. Excess stain was washed with water. The membrane was pre-hybridized for 6-8 hrs at 65°C in buffer containing 5X SSC, 5 X Denhardt reagent (0.5% Ficoll, 0.5% PVP, 0.5% BSA) 0.1 % SDS and 100μg/ml denatured salmon sperm DNA and 10% dextran sulphate. Thereafter, to the prehybridization solution radiolabelled probe (pre-denatured by boiling for 10min) was added. After 16-18 hrs of incubation. at 65°C in the hybridization solution, the membrane was washed twice with 2 X SSC, 0.1% SDS at room temperature for 5 min, twice with 0.5 X SSC, 0.1 % SDS at 65°C for 15 min and twice with 2 X SSC at room temperature for 5 min. The membrane was then exposed to X-ray film for autoradiography.

Total Protein Isolation from Plants and Estimation: Leaves were homogenized in liquid nitrogen and were grinded in mortar and pestle in extraction buffer consisting of 56 mM $Na_2CO_3$, 5mM DTT, 10mM Isoascorbate, 12% Sucrose, 2mM EDTA, and 2% SDS. The samples were vortexed thoroughly. They were incubated at 80°C for 20 min and were centrifuged at 13000 rpm for 5 min, and supernatant was collected. To the supernatant ice cold acetone (final concentration should be 90%) containing 0.1%v/v b-mercaptoethanol was added, mixed properly and kept in 20°C for 1 hour. Pellet done by centrifugation (13000 rpm for 10 min). The pellet was dissolved in 20μl of NaOH (0.1 M) and protein estimation was done with Bradford's method.

Isolation of Broken Plastids: Isolation of broken plastids were done as described by Tripathy and Mohanty, 1980. Around 5 g of leaves were homogenized in 40 ml of isolation buffer containing 0.4 M sorbitol, 0.05 M Hepes/KOH (pH 7.3), 1 mM $MgCl_2$, 1mM EDTA at 4°C and in green safe light Homogenae was passed trough 8 layers of cheesecloth and 1 layer of miracloth and centrifuged at 4000 rpm for 7 min. Pellet containing broken plastids was suspended in suspension buffer containing sorbitol 0.4 M, tris 0.05 M pH 7.5, $MgCl_2$ 1 mM and EDTA 1 mM.

Polyacrylamide Gel Electrophoresis of Proteins (SDS-PAGE): SDS-PAGE was performed according to Laemmli, 1970. Gels were prepared and electrophoresed under reducing and denaturing conditions in presence of β-ME and SDS. Protein samples were prepared by mixing ¼ volume of 4X sample buffer (200 mM Tris-Cl, pH 6.8,2% SDS, 40% glycerol, 8% β-ME, 0.04% bromophenol blue). The samples were boiled for 3 min in a water bath and the thylakoid samples were incubated at 50°C for 1h, and then loaded onto gel. Until the proteins get stacked, the gel was electrophoresed at low voltage (120V). Gels were stained with CBB (0.25% Coomassie brilliant blue R-250, 50% methanol and 10% acetic acid).

Table 4: Composition of 12% resolving & 3.5% stacking.

| Reagent | Resolving (10 ml) | Stacking (5 ml) |
|---|---|---|
| Acrylamide stock solution (30%) | 4.0 ml | 0.83 ml |
| 4X Resolving Gel buffer (1.5 M Tris Cl, pH 8.8) | 2.5 ml | - |
| 4X Stacking Gel buffer (0.5 M Tris Cl pH 6.8) | - | 1.3 Ml |
| Water | 3.3 ml | 3.5 ml |
| Ammonium per sulphate 10% | 0.1 ml | 0.05 ml |
| TEMED | 0.004 ml | 0.005 ml |

Western Blotting: Western blotting was done according to the method described in Sambrook et al., 1989. Mini trans blot Electrophoretic Cell (ATTO Co., Japan) was used to transfer the proteins from the gel onto nitrocellulose membrane. The apparatus for electro blotting was assembled according to the manufacturer's instruction. Electro blotting was performed in the presence of 39 mM glycine, 48 mM Tris base, 0.037% SDS and 20% methanol at a constant voltage (V) for 1 hrs. After the transfer was over, gel-facing side of the membrane was marked and the membrane was stained in ponceau S (0.1% ponceau S in 1% acetic acid). Markers were marked and the membrane was rinsed briefly in TBST (10 mM Tris, 150 mM NaCl, pH 7.5 and 0.05% Tween -20) and then incubated in blocking solution (4% BSA in TBST) for 1 hr with gentle shaking. The blocking solution was replaced with primary antibody solution (antibody in TBST containing 1% BSA) and incubation was continued for another 1 hr with gentle shaking at room temperature. Thereafter, the blots were washed thrice with TBST for 5 min each. After washing, the blots were transferred to alkaline-phosphate conjugated secondary antibody solution (1:15, 000 dilution in TBST containing 1% BSA) and further incubated for 1hr. The protein antibody complex was developed in 0.1 M Tris-HC1, pH 9.5, 0.1 M NaCl, 5mM $MgC1_2$ containing 150 µg/ml of NBT, and 75 µg/ml BCIP. The developing reaction was stopped by adding 10 mM EDTA, pH 8.0.

TRANSMISSION ELECTRON MICROSCOPY: Four-day-old etiolated seedlings of both wild type and porC over expressed Arabidopsis thaliana seedlings were taken and vacuum infiltrated with 2.5% glutaraldehyde solution for 30 minutes. After that the seedlings were kept for overnight in the same solution for fixation (Sabatini et al. 1963). The tissues were spinned down and 0.1M sodium-phosphate buffer (pH 7.0) was added. After this tissue was kept in secondary fixative containing 1% $OsO_4$. Fixation was done by immersing the tissue in fixative at 4°C for 2-4 h. After this tissue was washed with 0.1M $PO_4$ buffer, dehydration was done, which involved following steps:

1) 30% acetone twice for 15 minutes each time.
2) 50% acetone twice for 15 minutes each time.
3) 70% acetone twice for 15 minutes each time.
4) 80% acetone twice for 15 minutes each time.
5) 90% acetone twice for 15 minutes each time.
6) 95% acetone twice for 15 minutes each time.
7) Dry acetone twice for 15 minutes each time.
8) Dry acetone twice for 30 minutes each time.

All these steps were done at room temperature. For clearing tissues of acetone, epoxy propane or xylene was used twice for 30 minutes at room temperature. After this infiltration was done in a resin containing araldite and toluene in following ratios:

1) 1 part of araldite + 3 parts of toluene.
2) 2 parts of araldite + 2 parts of toluene.
3) 3 parts of araldite + 1 part of toluene.

For embedding araldite cy212 embedding medium was prepared. After embedding the liquid araldite was polymerized in a gradual process by keeping blocks at 50°C for 12-24 h and then at 60°C for 24-48 h. After this, sectioning was done using ultramicrotomes. These ultra thin sections were then stained in uranyl acetate. Saturated solution of uranyl acetate was prepared in 50% ethanol. This was mixed vigorously and centrifuged to allow the excess of uranyl acetate to settle down. For staining small amount of uranyl acetate was taken in a dean watch glass. Grid containing section was placed on to the stain. Staining was done in dark for 10-15 min. Each grid was then washed in 50% ethanol twice and distilled water twice with continuous agitation. This was then dried carefully on filter paper. After this sections were viewed in a Transmission Electron Microscope (Phillips CM-10).

Plant Growth Conditions: Both wild-type (WT) and transgenic sense plants of Arbidopsis thaliana plants were grown in growth chamber (Conviron, Canada) in a 14h/10h dark/light cycle. The light intensity was of 100 μmoles $m^{-2}$ $s^{-1}$ and the temperature set up was 22°C± 2°C.

Chlorophyll and Carotenoid Estimation: The extraction of Chlorophylls & Carotenoids from leaf tissues, from etiolated seedlings as well as from isolated chloroplasts were done under a dim, green safe light Leaf tissues (also seedlings) were homogenized in 90% chilled ammonical acetone (10ml) in a pre-chilled mortar and pestle. Three replicates were taken for each batch. Homogenate was centrifuged at 10,000 rpm for 10 min.

Supernatant was taken for estimating chl and carotenoid. Absorbance was taken at 663nm, 645nm and 470nm. Reference cuvette contained 90% amonical acetone. Chl was calculated as described by Porra et al., 1989 and carotenoids were calculated as described by Welbum and Lichenthaler, 1984.

$$\text{Chla} = (14.21 \times \text{OD663} - 3.01 \times \text{OD 645})\ \text{V/W}$$

$$\text{Chlb} = (25.23 \times \text{OD 645} - 5.16 \times \text{OD 663})\ \text{V/W}$$

$$\text{Chl (a+b)} = 9.05 \times \text{OD 663} + 22.2 \times \text{OD 645})\ \text{V/W}$$

$$\text{Carotenoids} = (1000 \times \text{OD 470} - \{3.27 \times \text{Chla} - 1.04 \times \text{Chlb}\}/5)\ \text{V/227} \times \text{W.}$$

Extraction and Determination of Tetrapyrroles: Total chlorophyll pigments were isolated as described before by grinding the different tissues with 90% ammonical acetone. Fully esterfied tetrapyrroles were extracted by adding equal volume of hexane to it. While the mono-and dicarboxylic tetrapyrroles remained in the HEAR phase, the fully esterified tetrapyrroles were transferred to hexane phase. Quantitative estimation of Pchlide, Mg-Proto and Mpe were carried out spectrofluorometrically using the HEAR (Hukmani and Tripathy, 1992).

Light and ALA Treatment: The Arabidopsis seedlings grown for four days in complete dark were transferred to different light intensity of 10 μmoles $m^{-2}$ $s^{-1}$, 40 μmoles $m^{-2}$ $s^{-1}$, 250 μmoles $m^{-2}$ $s^{-1}$ and continuous far red (cFR) light (50 μmoles $m^{-2}$ $s^{-1}$). Light intensity was measured with a quantum sensor (LI-COR, USA). Both wild type and porC over expressed plants were grown in growth chamber at 22°C under 100 μmoles $m^{-2}$ $s^{-1}$ of light intensity with 14h/10h dark/light cycle.

For ALA treatment, both wild type and transgenic plants were grown in pot having agropeat soil for five weeks. Each pot having 4 Arabidopsis plants was sprayed with 15 ml of aqueous ALA solution (3mM, pH 4.8). The plants along with their pots were immediately wrapped in aluminum foil to exclude light and to prevent the rapid drying of the solution. The control seedlings/plants were sprayed with distilled water (pH 4.8). The pots were then kept in dark room for 14 h before exposing to light (70 μmoles $m^{-2}$ $s^{1}$).

Net synthesis of Pchlide: To empty the endogenous Pchlide pool Arabidopsis plants grown under 100 μmoles $m^{-2}$ $s^{-1}$ and 14/8h light dark photoperiod were exposed to cool-white fluorescent light (70 μmoles $m^{-2}$ $s^{1}$) for 10 minutes and leaves were excised immediately after the light treatment, weighed and homogenized in 90% ammonical acetone. After light exposure, plants were sprayed with ALA and water, kept in dark and leaves were harvested after 3 hours. Three replicates of each sample were hand homogenized in 10 ml of ice cold 90% ammonical acetone at 4°C and homogenate was centrifuged at 10,000rpm for 10 min. Supernatant was taken for the HEAR preparation and accumulation of Pchlide was measured spectrofluorometrically (Tewari and Tripathy, 1998). The net synthesis of Pchlide was calculated by deducing the Pchlide value after 10 min of light exposure from those recorded after 3 h dark interval.

Protochlorophyllide Oxidoreductase Activity: Four weeks grown Arabidopsis plants (under 100 μmoles $m^{-2}$ $s^{-1}$ and 14/8h light dark photoperiod) sprayed with ALA and distilled water (without ALA) were transferred to complete dark and incubated for 6 hours. After which leaves were harvested, homogenized in 90% ammonical acetone in dark. Subsequently, plants were exposed to cool-white fluorescent light (70 μmoles $m^{-2}$ $s^{-1}$) for 15 minutes and leaves were harvested, weighed and homogenized in 90% ammonical acetone in light. Acetone tissue homogenates were centrifuged at 10,000 rpm in SS-34 rotor for 10 min at 4°C and from supernatant HEAR was prepared. Pchlide contents of Arabidopsis leaves before and after light exposure, were estimated from HEAR fraction by spectrofluoremetry. Three replicates were taken for each data points. The percent phototransformation was calculated as: [(Pchlide content before phototransformation - pchlide content after phototransformation)/ Pchlide content before phototransformation] x 100.

Anthocyanin Measurement: The anthocyanin estimation was carried out according to Feinbaum and Ausubel, 1988. Leaves were harvested from both wild and over expressed plants, cut into pieces, their fresh weight measured and anthocyanins extracted overnight in 3ml 1% acidic methanol (1% HCL in methanol). Phase partitioning was done next day by adding 3 ml chloroform and 2 ml distilled water. Absorbance of aqueous phase was recorded at 530 and 657nm. The total amount of anthocyanin ($A_{530}$-$A_{657}$) was estimated and normalized to per gram fresh weight.

Estimation of lipid peroxide: The level of lipid peroxidation products were estimated using 200 mg fresh tissue according to the method of Hodges et a!., 1999 and were expressed as thiobarbituric acid reactive substances (TBARS). Plant samples were extracted in 0.25% 2-thiobarbituric acid (TBA) in 10% TCA using a mortar and pestle. Contents were heated at 95°C for 30 minutes and the quickly cooled in an ice bath and centrifuged at 12,000 rpm for 10 min. The absorbance of the supernatant was read at 532 nm and correction for unspecific turbidity was done by subtracting the absorbance of the same at 600nm. The blank was 0.25% TBA in 10% TCA. The concentration of lipid peroxides together with oxidatively modified proteins were quantified and expressed as total TBARS in terms of nmol $g^{-1}$ fresh weight using an extinction coefficient of 155 $nM^{-1}$ $cm^{-1}$. TBARS are an index of lipid peroxidation.

To increase the accuracy of determining TBA-MDA levels by correcting for compounds other than MDA, which absorb at 532 nm, subtraction of the absorbance at 532 nm of a solution containing plant extract incubated without TBA from an identical solution containing TBA was done. A 1-ml aliquot of appropriately diluted sample was added to a test tube with 1 m of either (i) TBA solution comprised of 20.0% (w/v) trichloroacetic acid (-TBA) or (ii) TBA solution containing the above plus 0.65% TBA (+TBA). Samples were then mixed vigorously, heated at 95°C in a water bath for 25 min, cooled, and centrifuged at 12,000 rpm for 10 min. Absorbances were read (Shimadzu, Japan) at 440 nm, 532 nm, and 600 nm. Malondialdehyde equivalents were calculated in the following manner 1) ((Abs $532_{+TBA}$)-(Abs $600_{+TBA}$)-(Abs $532_{-TBA}$)-(Abs$600_{-TBA}$)]=A; 2) [(Abs $440_{+TBA}$-Abs $600_{+TBA}$)0.0571]=B;3) MDA equivalents. (nmol.$ml^{-1}$)=(A-B/157 000) $10^6$.

Electrolyte Leakage: Leaf discs ( 12 in number, 8mm diameter) from both control and treated plants were taken by using a cork borer and rinsed briefly with distilled water. The discs were dried on filter paper and kept in petriplates containing 20ml-deionised water. Petriplates were placed on to and fro shaker (80rpm) for 4h. After specific interval of time the extent of solute leakage (Initial Conductivity, IC) was monitored with a conductivity meter, which was calibrated against 0.01 N KCL solution. The readings were corrected for the cell constant C, which was calculated according to the following formula.

$$C = \frac{0.001413 \times R\ (KCL)}{1+0.02\ (t-25)}$$

where, R= 1.412μmohs $cm^{-2}$ and t=27°C

Samples collected at different time periods were then boiled for 10 minutes and allowed to reach the room temperature, while placed on shaker to facilitate total electrolyte leakage from the tissues. Volume was then made to 20 ml with deionised water and shaken for another 5 minutes. Total electrolyte leakage (Final Conductivity, FC) was then recorded. The percentage of electrolyte leakage was calculated by using the formula: (IC/FC) x 100.

Reactive Oxygen Species (ROS) Staining: Detection of $H_2O_2$ was done by endogenous peroxidase-dependent insitu histochemical staining using, 3,3-diamino benzidine (DAB) (Thordal-Christensen et al., 1997) and detection of super oxide radicals ($O_2^-$) was done by imaging of purple formazon deposits (Flohe and Otting, 1984), which result from the reaction of nitroblue tetrazolium (NBT) with super oxide. Both wild type and porC transgenic Arabidopsis leaves were cut from the petiole, submerged into wells containing 6-8ml DAB [(1 mg/ml), DAB prepared in 10mM MES, pH 6.5] and NBT [(1 mg/ml), NBT prepared in 50mM potassium phosphate buffer, pH 6.4] solution. Then these were vacuum infiltrated for 20 minutes in dark. After infiltration leaves were briefly rinsed in distilled water twice and transferred into falcon tubes containing ethanol (96%), with holes in the cap. Falcon tubes were allowed to float into boiling water until chlorophyll was completely removed from leaves. Leaves were then transferred in fresh ethanol (96%) and kept for 2-3 days at room temperature.

Singlet $O_2$ ($^1O_2$) Measurement: The production of $^1O_2$ was determined in terms of RNO bleaching using a spectrophotometric method as described by Joshi and Pathak, 1984. RNO was used for spectrophotometric assay and histidine was used as a trap of $^1O_2$. Chloroplast was isolated in complete dark. Chloroplasts (100μg Chl $ml^{-1}$) were

incubated with RNO (300μg $ml^{-1}$) and histidine (10 mM) In one eppendorf tube in dark and another tube was illuminated in Hansatech oxygen evolution chamber for different time period during the assay. Samples were taken after various time intervals. After centrifugation (12000 rpm for 15 min), the supernatant was decanted and the absorbance was read at 440 nm in a spectrophotometer (Shimadzu, UV160). The dark samples were used as blank.

Fluorescence spectra of Chloroplasts: The room temperature and low temperature (77k) fluorescence emission spectra of chloroplasts were recorded in SLM-AMINCO 8000C spectroflurometer. The slit widths of excitation and emission were 4nm. For room temperature emission spectra, chloroplasts equivalent to 5 μg Chl $ml^{-1}$ were suspended in 25 mM hepes-NaoH buffer (pH 7.6). The 77k Chl a emission experiments were done with low temperature accessory and chloroplast were suspended in 25 mM hepes-NaoH buffer (pH 7.6) containing 50% glycerol at a concentration of 5 μg Chl $ml^{-1}$. The spectra were corrected for photomultiplier sensitivity.

Shibata Shift: To study the Shibata shift the plastids were isolated from Arabidopsis plants grown in complete dark for 4 days. The plastid suspensions were given a saturating pulse of red light (1500 μmoles $m^{-2}$ $s^1$, 0.1 Sec) and then incubated for different time periods up to 10 minutes in dark. Low temperature spectra were recorded before red light flash, immediately after red light flash, and after different dark incubation from 600-770nm. All the operations were carried out in green, safe light.

Pulse Amplitude Modulation Measurement (PAM): $F_0$, Fv/Fm, Electron transport rate (ETR), Photochemical quenching (qP), non-photochemical quenching (qN) and Photo system II quantum yield ($\Phi_{11}$) were measured at room temperature by a PAM-2100 Chl fluorometer (Walz, Germany). Either leave or whole plant was taken as the experimental material. During the time of experiment red actinic illumination (wavelength, 655 nm) provided by five LEDS (H3000) (Stanley, CA, USA) focused onto the leaf surface (79 $mm^2$). Two other H-3000 LEDS, that emit 650-nm pulses, were used as measuring light. Leaf clip holder 2030-B, equipped with a microquantum sensor, monitored photosynthetically active radiation (PAR). An additional spacer was added to the clip in order to avoid pinching of the leaf. Chl fluorescence was detected by a photodiode (BYP 12; Siemens, Munich, Germany) that was shielded by a long-pass far-red filter (RG9; Schott, Southbidge, MA, USA) and a heat filter. All the PAM-2100 fluorescence data were recorded in a time span of 5 min and 20 s. The rate of acquisition by the DA-2100 software in the preset program used (listed as run 3 in the PAM-2100 handbook) was 10 ms $pulse^{-1}$. Run 3 measures Chl a fluorescence while calculating values of $q^N$ (a measure of NPQ [(Fm-Fm')/(Fm-$F_0$)]), qP (photochemical quenching of fluorescence [(Fm'-F)/Fm'-$F_0$)]), and Y (the photochemical yield [(Fm'-F)/Fm']. Recording of data including determination of $F_0$ (minimal fluorescence yield of dark-adapted sample) and Fm (maximal fluorescence of dark adapted sample). (Govindjee and Paul Spilotro, 2002) began 15 s following the onset of run 3.

## Claims

**1.** A method of increasing stress resistance in a plant comprising a process for the construction of a plant transformation vector comprising:

digesting a binary vector pCAMBIA1304 with EcoRI and Sal I;
subjecting the digested binary vector to the step of end filling by Klenow;
re-ligating the said binary vector by a known method;
obtaining CaMV 35 s promoter cassette with Ω enhancer from pSH9;
incorporating the said Camv35S prometer cassette into the Hind III site of the said binary vector to obtain modified pCAMBIA 1304 vector;
subjecting porC cDNA fragment (1206 bp) to the step of amplification using a pair of primers;
introducing EcoRI restriction sites to both the primers;
ligating the said amplified cDNA fragment to pGEM T-easy;
extracting EcoRI digested porC cDNA fragment from cloned pGEM T-easy;
inserting said EcoRI digested porC cDNA fragment into modified pCAMBIA 1304 plant transformation vector to produce recombinant plasmids construct (pCAMBIA 1304-AtporC);
wherein for the step of amplification of a porC cDNA fragment (1206 bp), cDNA library of *Arabidopsis thaliana* is used; and
wherein polymerase chain reaction (PCR) was amplified with a pair of primers: forward primer 5'-tctagagaattcatggctctccaagctgcctattctc-3' and reverse primer 5'-gaattctcatgccaaaccaacaagcttctcgc-3', designed based on the cDNA sequence.

**2.** The method as claimed in claim 1, wherein the step of amplification of cDNA was carried out using Taq DNA polymerase and a set of convergent primers.

**3.** The method as claimed in claim 1, wherein the DNA fragments were ligated to vectors by using T4 DNA ligase overnight at 4°C or 16°C.

**4.** The method as claimed in claim 1, further comprising the step of overexpressing the porC gene in the plant to make the plant highly resistant to biotic and abiotic stresses.

## Patentansprüche

**1.** Verfahren zum Erhöhen der Stressresistenz in einer Pflanze, umfassend einen Prozess zur Konstruktion eines Pflanzen-Transformationsvektors, umfassend:

Verdauen eines binären pCAMBIA-1304-Vektors mit EcoRI und Sal I,
Unterziehen des verdauten binären Vektors dem Schritt des Auffüllens von Enden nach Klenow,
Neuligieren des binären Vektors mit einem bekannten Verfahren,
Erhalten einer CaMV-35s-Promotorkassette mit Ω-Enhancer aus pSH9,
Einarbeiten der CaMV-35s-Promotorkassette in die HindIII-Stelle des binären Vektors, um modifizierten pCAMBIA-1304-Vektor zu erhalten,
Unterziehen von porC-cDNA-Fragment (1206 bp) dem Schritt der Amplifikation unter Verwendung eines Primer-Paares,
Einbringen von EcoRI-Restriktionsstellen in beide Primer,
Ligieren des amplifizierten cDNA-Fragments mit pGEM-T Easy,
Extrahieren von mit EcoRI verdautem porC-cDNA-Fragment aus geklontem pGEM-T Easy,
Einsetzen des mit EcoRI verdauten porC-cDNA-Fragments in den modifizierten pCAMBIA-1304-PflanzenTransformationsvektor, um rekombinante Plasmidkonstrukte (pCAMBIA-1304-AtporC) zu erzeugen,
wobei für den Schritt des Amplifizierens eines porC-cDNA-Fragments (1206 bp) die cDNA-Bibliothek von *Arabidopsis thaliana* verwendet wird, und
wobei die Polymerase-Kettenreaktion (PCR) mit einem Primer-Paar amplifiziert wurde: dem Vorwärts-Primer 5'-tctagagaattcatggctctccaagctgcctattctc-3' und dem Rückwärts-Primer 5'-gaattctcatgccaaaccaacaagcttctcgc-3', entwickelt auf der Grundlage der cDNA-Sequenz.

2. Verfahren nach Anspruch 1, wobei der Schritt des Amplifizierens von cDNA unter Verwendung von Taq-DNA-Polymerase und einem Satz konvergenter Primer ausgeführt wurde.

3. Verfahren nach Anspruch 1, wobei die DNA-Fragmente unter Verwendung von T4-DNA-Ligase über Nacht bei 4 °C oder 16 °C an Vektoren ligiert wurden.

4. Verfahren nach Anspruch 1, ferner den Schritt des Überexprimierens des porC-Gens in der Pflanze umfassend, um die Pflanze hochgradig resistent gegen biotischen und abiotischen Stress zu machen.

## Revendications

1. Procédé pour augmenter la résistance au stress d'une plante, comprenant un procédé de construction d'un vecteur de transformation végétal consistant à :

faire digérer un vecteur binaire pCAMBIA1304 par EcoRI et Sal I ;
soumettre le vecteur binaire digéré à l'étape de remplissage terminale par Klenow ;
religaturer ledit vecteur binaire par un procédé connu ;
obtenir une cassette promoteur CaMV35S par l'amplificateur Ω à partir de pSH9 ;
incorporer ladite cassette promoteur CaMV35S dans le site Hind III dudit vecteur binaire pour obtenir le vecteur pCAMBIA1304 modifié ;
soumettre un fragment d'ADNc porC (1206 pb) à l'étape d'amplification à l'aide d'une paire d'amorces ;
introduire les sites de restriction EcoRI aux deux amorces ;
ligaturer ledit fragment d'ADNc amplifié à pGEM T-easy ;
extraire le fragment d'ADNc porC digéré par EcoRI à partir de pGEM T-easy cloné ;
insérer ledit fragment d'ADNc porC digéré par EcoRI dans le vecteur de transformation végétal pCAMBIA1304 modifié pour obtenir un produit de construction de plasmides recombinants (pCAMBIA1304-AtporC) ;
où pour l'étape d'amplification d'un fragment d'ADNc porC (1206 pb), la banque d'ADNc d'*Arabidopsis thaliana* est utilisée ; et
où la réaction en chaîne par polymérase (PCR) a été amplifiée avec une paire d'amorces : amorce sens 5'-TCTAGAGAATTCATGGCTCTCCAAGCTGCCTATTCTC-3' et amorce antisens 5'-GAATTCTCATGCCAAACCAACAAGCTTCTCGC-3', conçues selon la séquence d'ADNc.

2. Procédé selon la revendication 1, dans lequel l'étape d'amplification de l'ADNc a été réalisée au moyen de l'ADN polymérase Taq et un ensemble d'amorces convergentes.

3. Procédé selon la revendication 1, dans lequel les fragments d'ADN sont ligaturés à des vecteurs au moyen de l'ADN ligase T4 durant toute une nuit à 4 °C ou 16 °C.

**4.** Procédé selon la revendication 1, comprenant en outre l'étape de surexpression du gène porC dans la plante pour rendre la plante hautement résistante aux stress biotiques et abiotiques.

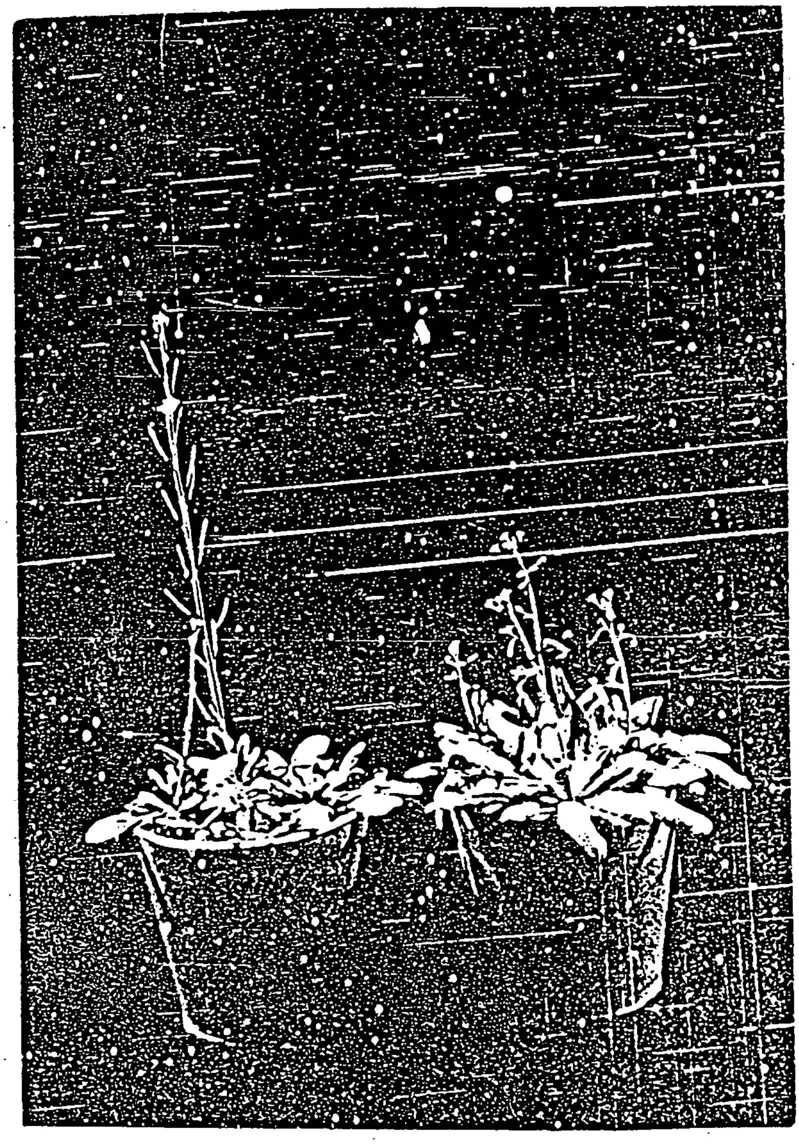

Fig. 1

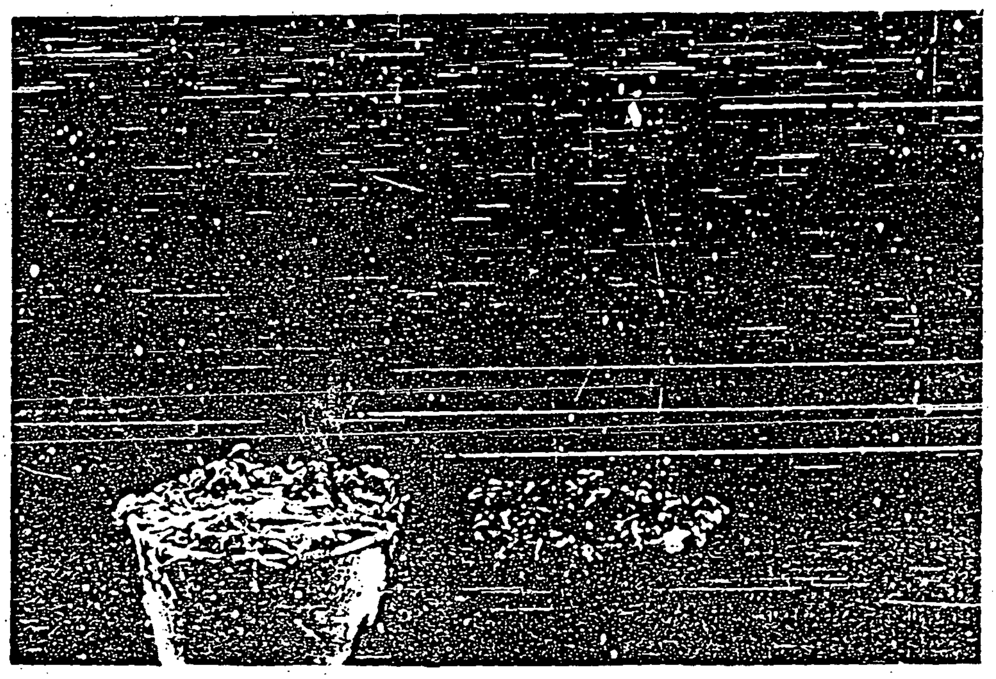

Fig. 2

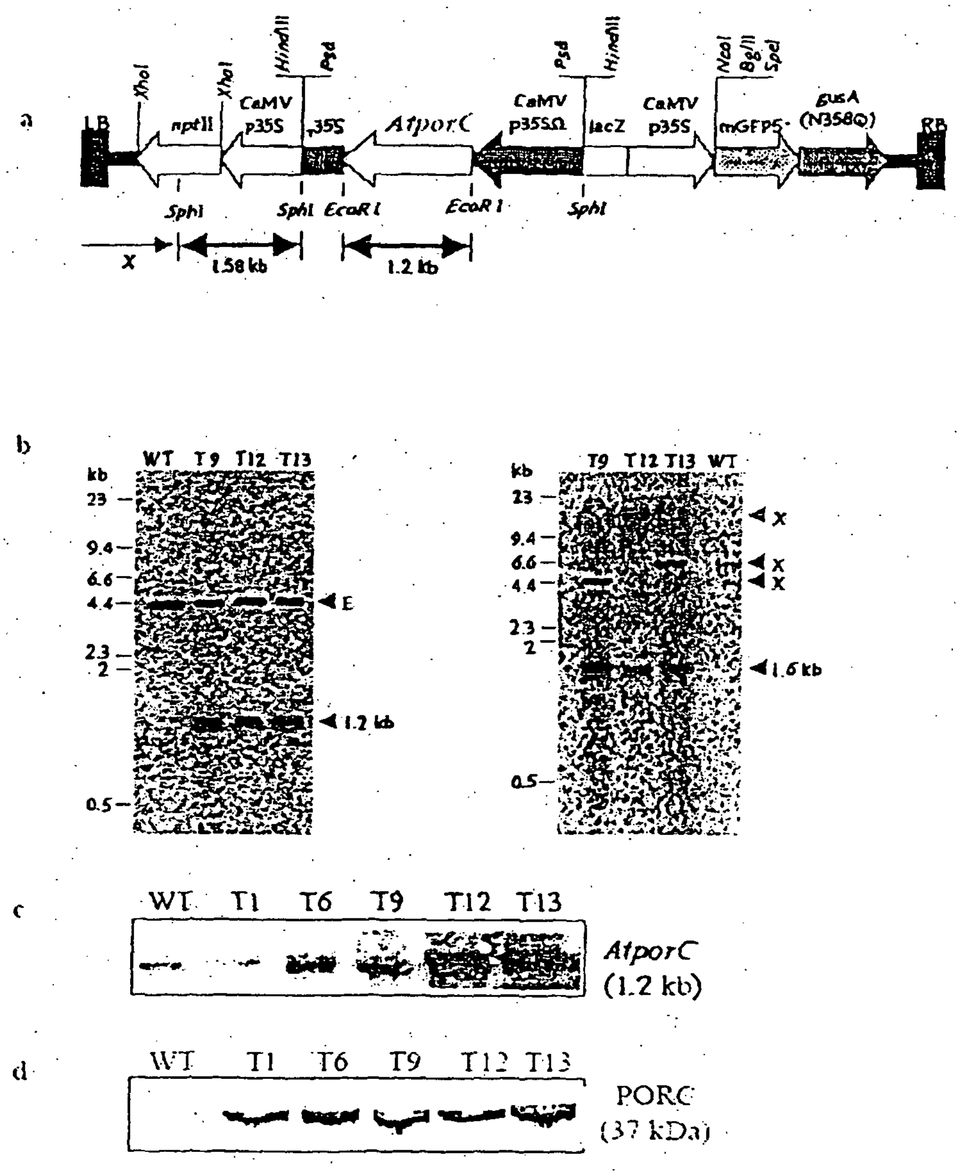
a
LB
XhoI
nptII
XhoI
CaMV p35S
HindIII
PstI
T35S
AtporC
CaMV p35SΩ
PstI
HindIII
lacZ
CaMV p35S
NcoI
BglII
SpeI
mGFP5
gusA (N358Q)
RB
SphI
SphI
EcoR I
EcoR I
SphI
X
1.58 kb
1.2 kb
b
WT T9 T12 T13
kb
23
9.4
6.6
4.4
2.3
2
0.5
E
1.2 kb
T9 T12 T13 WT
X
X
X
1.6 kb
c
WT T1 T6 T9 T12 T13
AtporC (1.2 kb)
d
WT T1 T6 T9 T12 T13
PORC (37 kDa)

Fig. 3

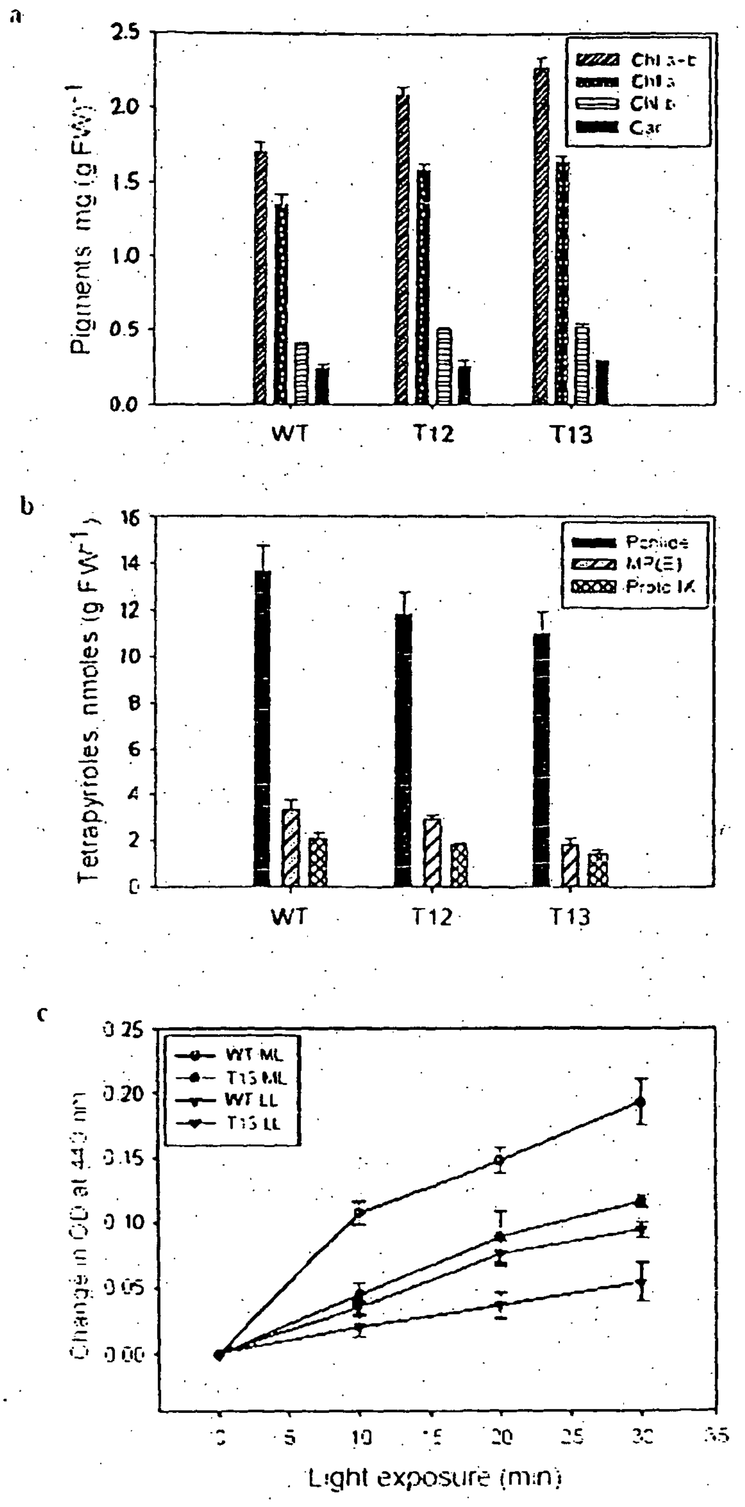
a
Pigments mg (g FW)-1
2.5
2.0
1.5
1.0
0.5
0.0
WT
T12
T13
b
16
14
12
10
8
6
4
2
WT
T12
T13
c
0.25
0.20
0.15
0.10
0.05
0.00
WT ML
WT LL
10
20
Light exposure (min)

Fig 4

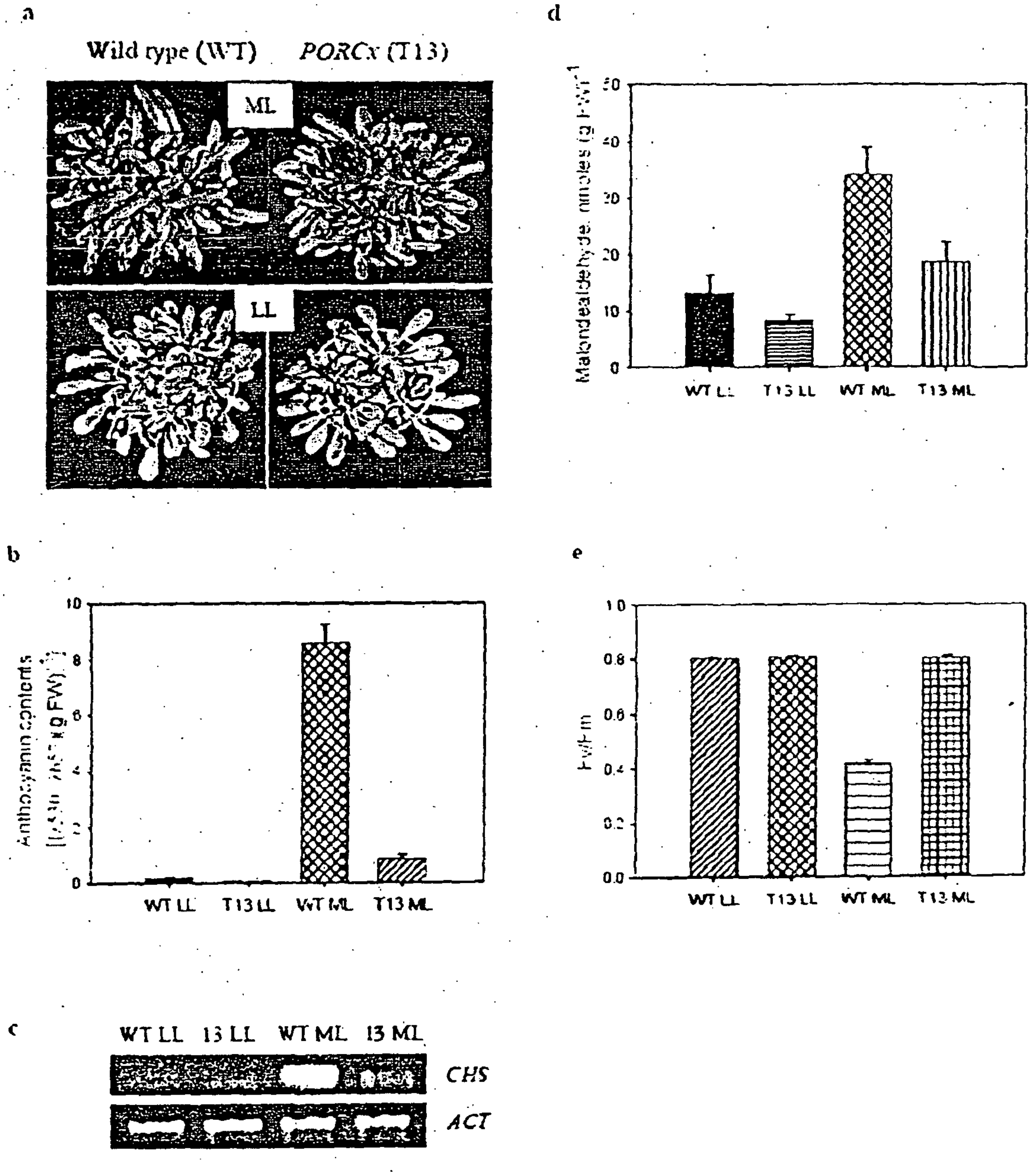
a
Wild type (WT)
PORCx (T13)
ML
LL
b
Anthocyanin contents
WT LL
T13 LL
WT ML
T13 ML
c
WT LL
13 LL
WT ML
13 ML
CHS
ACT
d
Malondealdehyde, nmoles (g FW)$^{-1}$
WT LL
T13 LL
WT ML
T13 ML
e
Fv/Fm
WT LL
T13 LL
WT ML
T13 ML

Fig. 5

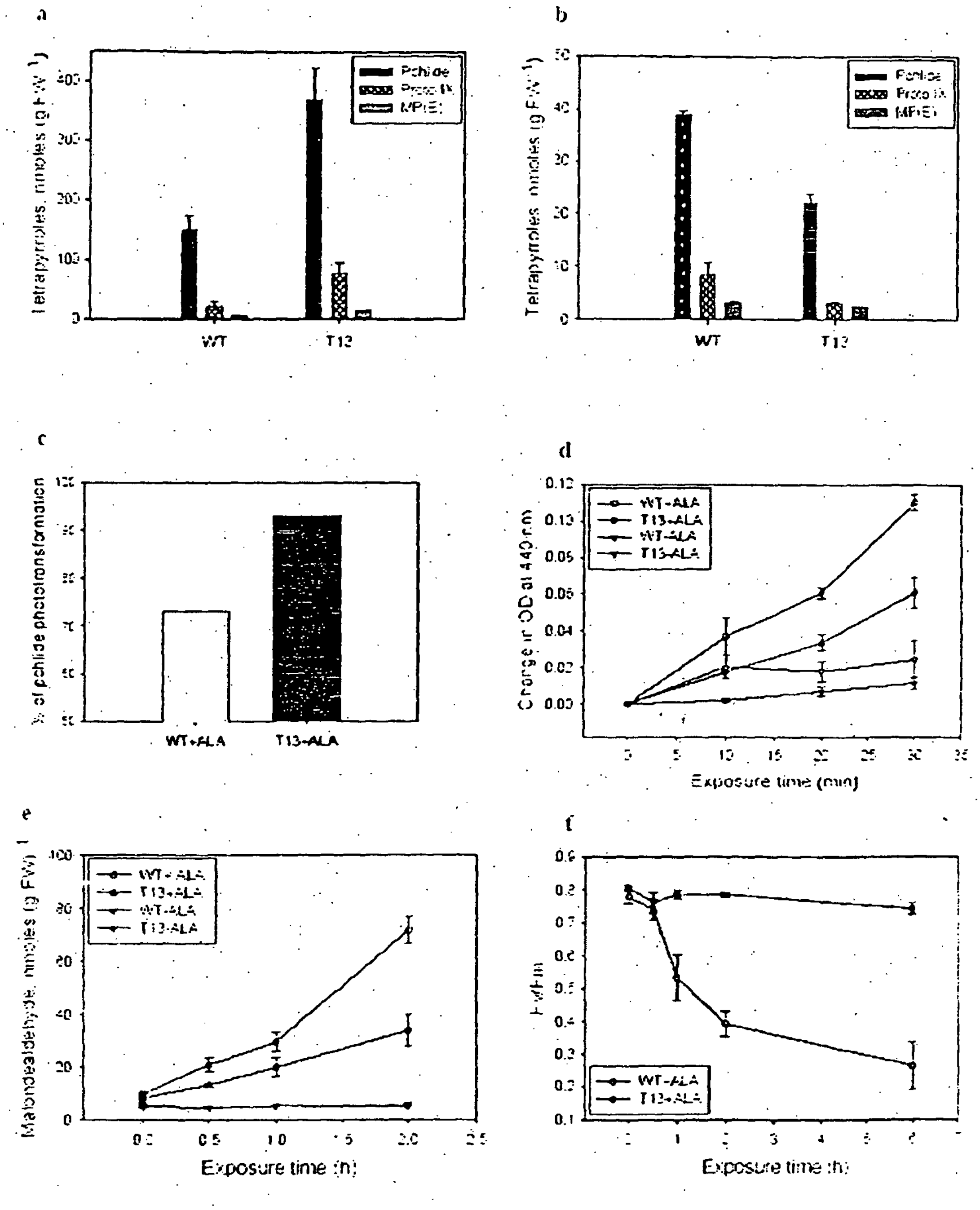
a
Tetrapyrroles, nmoles (g FW⁻¹)
Pchlide
Proto IX
MP(E)
WT
T13
b
Tetrapyrroles, nmoles (g FW⁻¹)
Pchlide
Proto IX
MP(E)
WT
T13
c
% of pchlide phototransformation
WT+ALA
T13+ALA
d
Change in OD at 440 nm
WT+ALA
T13+ALA
WT-ALA
T13-ALA
Exposure time (min)
e
Malondealdehyde, nmoles (g FW)⁻¹
WT+ALA
T13+ALA
WT-ALA
T13-ALA
Exposure time (h)
f
Fv/Fm
WT+ALA
T13+ALA
Exposure time (h)

Fig. 5 (CONT'D)

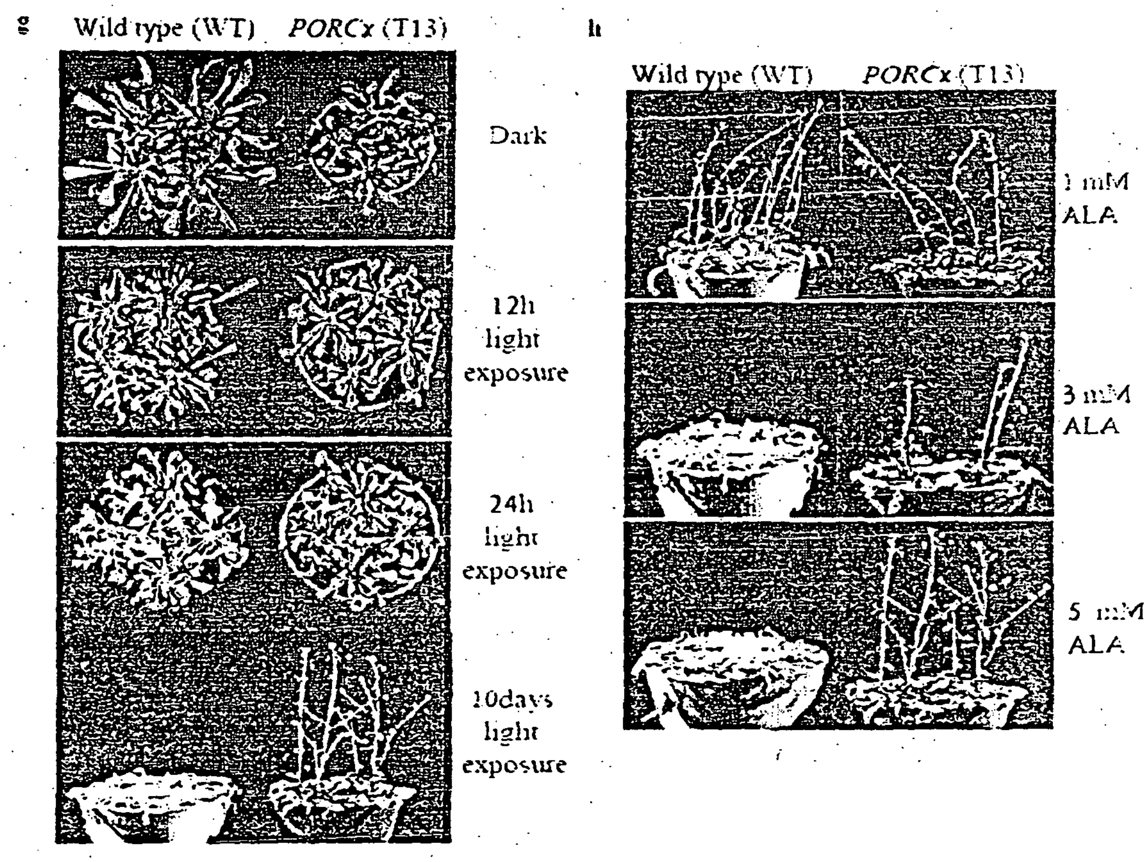
g
Wild type (WT)
PORCx (T13)
Dark
12h light exposure
24h light exposure
10days light exposure
h
Wild type (WT)
PORCx (T13)
1 mM ALA
3 mM ALA
5 mM ALA

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 2007000308 W
- IN 2006DE01843

SEQUENCE LISTING

<110> DEPARTMENT OF BIOTECHNOLOGY and JAWAHARLAL NEHRU UNIVERSITY

<120> RESISTANCE OF PLANTS TO BIOTIC AND ABIOTIC STRESSES BY OVEREXPRESSION OF PROTOCHLOROPHYLLIDE OXIDOREDUCTASE C AND ITS ISOFORMS

<130> SXT/P117041EP

<140> PCT/IN2007/000308
<141> 2007-07-24

<150> IN2006DE01843
<151> 2006-08-17

<160> 47

<170> PatentIn version 3.3

<210> 1
<211> 37
<212> DNA
<213> Arabidopsis sp.

<400> 1

tctagagaat tcatggctct ccaagctgcc tattctg 37

<210> 2
<211> 32
<212> DNA
<213> Arabidopsis sp.

<400> 2
gaattctcat gccaaaccaa caagcttctc gc 32

<210> 3
<211> 34
<212> DNA
<213> Arabidopsis sp.

<400> 3
ggggatccca gacagttaca gccacgccgc cggc 34

<210> 4
<211> 24
<212> DNA
<213> Arabidopsis sp.

<400> 4
gggaattctc aaaccatctt agag 24

<210> 5
<211> 30
<212> DNA
<213> Arabidopsis sp.

<400> 5
cgggatcccg ctcagtcttc tttgctccgg 30

<210> 6
<211> 36
<212> DNA
<213> Arabidopsis sp.

<400> 6
tctagagaat tcatggccct tcaagcttct ttggtc 36

<210> 7
<211> 30
<212> DNA
<213> Arabidopsis sp.

<400> 7
gaattcttag gccaagccca cgagcttctc 30

<210> 8
<211> 25
<212> DNA
<213> Arabidopsis sp.

<400> 8
gggaattcgc ctccattacc gacca 25

<210> 9
<211> 25
<212> DNA
<213> Arabidopsis sp.

<400> 9
gggtcgacgc cgtccacgga ttttg 25

<210> 10
<211> 25
<212> DNA
<213> Arabidopsis sp.

<400> 10
gggaattcat ttcactttcg gagca 25

<210> 11
<211> 25
<212> DNA
<213> Arabidopsis sp.

<400> 11
gggtcgacgc ggtctaagga agatt 25

<210> 12
<211> 32
<212> DNA
<213> Arabidopsis sp.

<400> 12
gagctcgaga tgaacgccgc cgtgtttagt cc 32

<210> 13
<211> 27
<212> DNA
<213> Arabidopsis sp.

<400> 13
tcgaattcct tagccggaga aaggtag 27

<210> 14
<211> 33
<212> DNA
<213> Arabidopsis sp.

<400> 14
gagctcgaga tcaagagtca atggagacct ttg 33

<210> 15
<211> 21
<212> DNA
<213> Arabidopsis sp.

<400> 15
gagcgacaca gagaagtttg g 21

<210> 16
<211> 21

<212> DNA
<213> Arabidopsis sp.

<400> 16
cctactcagt accctctcag c 21

<210> 17
<211> 20
<212> DNA
<213> Arabidopsis sp.

<400> 17
ccggtgtgga tgttgtgagc 20

<210> 18
<211> 22
<212> DNA
<213> Arabidopsis sp.

<400> 18
agtatcatga atccggcttg tg 22

<210> 19
<211> 23
<212> DNA
<213> Arabidopsis sp.

<400> 19
gacacggcta aatcatctct aac 23

<210> 20
<211> 29
<212> DNA
<213> Arabidopsis sp.

<400> 20
cgggatcctg ttcagtggcc ggtggacca 29

<210> 21
<211> 23
<212> DNA
<213> Arabidopsis sp.

<400> 21
gggagtgtag aagaactatt agc 23

<210> 22
<211> 21
<212> DNA
<213> Arabidopsis sp.

<400> 22
gtggtgactt cgagcaccaa g 21

<210> 23
<211> 21
<212> DNA
<213> Arabidopsis sp.

<400> 23
gcacttgcag ctctcaaagg a 21

<210> 24
<211> 27
<212> DNA
<213> Arabidopsis sp.

<400> 24
ggctgcagtc agctgaaaat ctcggca 27

<210> 25
<211> 23
<212> DNA
<213> Arabidopsis sp.

<400> 25
gataccgaga acaagtttgt ttc 23

<210> 26
<211> 34
<212> DNA
<213> Arabidopsis sp.

<400> 26
cgggatccaa gctttcaaga attcttcaga tcag 34

<210> 27
<211> 23
<212> DNA
<213> Arabidopsis sp.

<400> 27
gtctcatgaa caccaatccc aac 23

<210> 28
<211> 30
<212> DNA
<213> Arabidopsis sp.

<400> 28
gggaattcgt cgacttatcg atcgatccct 30

<210> 29
<211> 20
<212> DNA
<213> Arabidopsis sp.

<400> 29
gcatggcgac gacggttaca 20

<210> 30
<211> 26
<212> DNA
<213> Arabidopsis sp.

<400> 30
gcttaaacac taagcttctc aatctc 26

<210> 31
<211> 20
<212> DNA
<213> Arabidopsis sp.

<400> 31
cccactatcc ttcgcaagac 20

<210> 32
<211> 31
<212> DNA
<213> Escherichia coli

<400> 32
cgcggatcca tgttaggttc gtagaaaccc c 31

<210> 33
<211> 33
<212> DNA
<213> Escherichia coli

<400> 33
gctcgagctc ccgggtcatt ggcctccctg ctg 33

<210> 34
<211> 21
<212> DNA
<213> Escherichia coli

<400> 34
atgcccacag gccgtcgagt t 21

<210> 35
<211> 26
<212> DNA
<213> Arabidopsis sp.

<400> 35
tcgaccatgg ggattgaaca agatgg 26

<210> 36
<211> 33
<212> DNA
<213> Arabidopsis sp.

<400> 36
attcgagctc tcagaagaac tcgtcaagaa ggc 33

<210> 37
<211> 21
<212> DNA
<213> Arabidopsis sp.

<400> 37
atggctgatg gtgaagacat t 21

<210> 38
<211> 22

<212> DNA
<213> Arabidopsis sp.

<400> 38
tcagaagcac ttcctgtgaa ca 22

<210> 39
<211> 24
<212> DNA
<213> Arabidopsis sp.

<400> 39
ttttttttt ttttttttt tttt 24

<210> 40
<211> 20
<212> DNA
<213> Arabidopsis sp.

<400> 40
atggtgatgg ctggtgcttc 20

<210> 41
<211> 22
<212> DNA
<213> Arabidopsis sp.

<400> 41
ttagagagga acgctgtgca ag 22

<210> 42
<211> 31
<212> DNA
<213> Arabidopsis sp.

<400> 42
ggcaagcttg gtataattgg gctcaaaaag g 31

<210> 43
<211> 27
<212> DNA
<213> Arabidopsis sp.

<400> 43
ggcaagcttg atgagggggg aaaaagt 27

<210> 44
<211> 29
<212> DNA
<213> Arabidopsis sp.

<400> 44
ggcaagcttg tagaagatga agagagctg 29

<210> 45
<211> 31
<212> DNA
<213> Arabidopsis sp.

<400> 45
gggggatcca tcaccgttga taggtgaact g 31

<210> 46
<211> 29
<212> DNA
<213> Arabidopsis sp.

<400> 46
ggcaagcttc agatcagttg agagttaac 29

<210> 47
<211> 31
<212> DNA
<213> Arabidopsis sp.

<400> 47
gggggatcct gttgtacgga actgaaggtg c 31